# FASCICULE DE BREVET EUROPEEN

(11) **EP 0 698 667 B1**
(45) Date de publication et mention de la délivrance du brevet: **16.06.2004**
(21) Numéro de dépôt: 95201976.8
(22) Date de dépôt: 18.07.1995
(51) Int. Cl.: C12N 15/56, C12N 9/24, A23L 3/34, D21C 3/00, C12N 1/21

(54) **Xylanase, microorganismes la produisant, molécules d'ADN, procédés de préparation de cette xylanase et utilisations de celle-ci**
Xylanase, Mikroorganismen zu deren Herstellung, DNA Moleküle, Verfahren zur Herstellung und Verwendungen dafür
Xylanase, microorganisms for its production, DNA molecules, process of preparation and use thereof

(30) Priorité: 26.07.1994 BE 9400706; 17.05.1995 BE 9500448
(43) Date de publication de la demande: 28.02.1996
(73) Titulaire: GENENCOR INTERNATIONAL, INC., Palo Alto, California 94304 (US)
(72) Inventeur: De Buyl, Eric, B-1630 Linkebeek (BE); Lahaye, Andrée, B-1080 Bruxelles (BE); Ledoux, Pierre, B-1200 Bruxelles (BE); Detroz, René, B-1328 Lasne (BE)
(74) Mandataire: Kiddle, Simon John

(56) Documents cités:
- EP-A- 0 634 490
- WO-A-94/01532
- WO-A-94/04664
- WO-A-95/18219
- BIOSCIENCE, BIOTECHNOLOGY, AND BIOCHEMISTRY, vol. 58, Janvier 1994 pages 78-81, NAKAMURA S. ET AL. 'Thermophilic alkaline xylanase from newly isolated alkaliphilic and thermophilic Bacillus sp. strain TAR-1'
- AGRICULTURAL AND BIOLOGICAL CHEMISTRY, vol. 49, 1985 TOKYO JP, pages 2033-2039, OKAZAKI W. ET AL. 'Purification and characterization of xylanases from alkalophilic thermophilic Bacillus spp.'
- APPLIED AND ENVIRONMENTAL MICROBIOLOGY, vol. 60, no. 3, Mars 1994 pages 763-770, IRWIN D. ET AL. 'Characterization and sequence of Thermomonospora fusca xylanase'
- JOURNAL OF MICROBIOLOGY AND BIOTECHNOLOGY, vol. 3, no. 3, 1993 pages 139-145, YU JU-HYUN ET AL. 'Nucleotide sequence and analysis of a xylanase gene (xynS) from alkali-tolerant Bacillus sp. YA-14 and comparison with other xylanases'
- FEBS LETTERS, vol. 171, no. 2, 1984 AMSTERDAM NL, pages 197-201, FUKUSAKI E. ET AL. 'The complete nucleotide sequence of the xylanase gene (xynA) of Bacillus pumilus'

### LISTE DE SEQUENCES

(1) INFORMATIONS GENERALES :
   (i) DEPOSANT :
      (A) NOM : SOLVAY (Société Anonyme)
      (B) RUE : rue du Prince Albert, 33
      (C) VILLE Bruxelles
      (E) PAYS : Belgique
      (F) CODE POSTAL : 1050
      (G) TELEPHONE : (02) 509.61.11
   (ii) TITRE DE L'INVENTION : xylanase, microorganismes la produisant, molécules d'ADN, procédés de préparation de cette xylanase et utilisations de celle-ci
   (iii) NOMBRE DE SEQUENCES : 29
   (iv) FORME LISIBLE PAR ORDINATEUR :
      (A) TYPE DE SUPPORT : Floppy disk
      (B) ORDINATEUR : IBM PC compatible
      (C) SYSTEME D'EXPLOITATION : PC-DOS:MS-DOS
(2) INFORMATION POUR LA SEQ ID NO:1:
   (i) CARACTERISTIQUES DE LA SEQUENCE :
      (A) LONGUEUR : 663 paires de bases
      (B) TYPE : acide nucléique
      (C) NOMBRE DE BRINS : simple
      (D) CONFIGURATION : linéaire
   (ii) TYPE DE MOLECULE : ADN génomique
   (xi) DESCRIPTION DE LA SEQUENCE: SEQ ID NO:1:
   CAAATCGTCA CCGACAATTC CATTGGCAAC CACGATGGCT ATGATTATGA ATTTTGGAAA     60
   GATAGCGGTG GCTCTGGGAC AATGATTCTC AATCATGGCG GTACGTTCAG TGCCCAATGG    120
   AACAATGTTA ACAACATATT ATTCCGTAAA GGTAAAAAAT TCAATGAAAC ACAAACACAC    180
   CAACAAGTTG GTAACATGTC CATAAACTAC GGAGCCAACT TCCAACCAAA TGGTAATGCG    240
   TATTTATGCG TCTATGGTTG GACTGTTGAC CCTCTTGTCG AATATTATAT TGTCGACAGT    300
   TGGGGCAACT GGCGTCCACC AGGAGCAACG CCTAAGGGGA CCATCACTGT TGATGGAGGA    360
   ACATATGATA TCTACGAGAC TCTTAGAGTC AATCAACCCT CCATTAAGGG GATTGCCACA    420
   TTTAAACAAT ATTGGAGTGT TCGAAGATCG AAACGCACGA GTGGCACGAT TTCTGTCAGC    480
   AACCACTTTA GAGCGTGGGA AAACTTAGGG ATGAATATGG GGAAAATGTA TGAAGTCGCG    540
   CTTACTGTAG AAGGCTATCA AAGTAGCGGA AGTGCTAATG TATATAGCAA TACACTAAGA    600
   ATTAACGGTA ACCCTCTCTC AACTATTAGT AATGACGAGA GCATAACTTT GGATAAAAAC    660
   AAT                                                                 663
(2) INFORMATION POUR LA SEQ ID NO:2:
   (i) CARACTERISTIQUES DE LA SEQUENCE :
      (A) LONGUEUR : 663 paires de bases
      (B) TYPE : acide nucléique
      (C) NOMBRE DE BRINS : simple
      (D) CONFIGURATION : linéaire
   (ii) TYPE DE MOLECULE : ADN génomique
   (xi) DESCRIPTION DE LA SEQUENCE: SEQ ID NO:2:
   CAA ATC GTC ACC GAC AAT TCC ATT GGC AAC CAC GAT GGC TAT GAT TAT      48
   Gln Ile Val Thr Asp Asn Ser Ile Gly Asn His Asp Gly Tyr Asp Tyr
    1               5                  10                  15
   GAA TTT TGG AAA GAT AGC GGT GGC TCT GGG ACA ATG ATT CTC AAT CAT      96
   Glu Phe Trp Lys Asp Ser Gly Gly Ser Gly Thr Met Ile Leu Asn His
               20                  25                  30
   GGC GGT ACG TTC AGT GCC CAA TGG AAC AAT GTT AAC AAC ATA TTA TTC     144
   Gly Gly Thr Phe Ser Ala Gln Trp Asn Asn Val Asn Asn Ile Leu Phe
           35                  40                  45
   CGT AAA GGT AAA AAA TTC AAT GAA ACA CAA ACA CAC CAA CAA GTT GGT     192
   Arg Lys Gly Lys Lys Phe Asn Glu Thr Gln Thr His Gln Gln Val Gly
       50                  55                  60
   AAC ATG TCC ATA AAC TAC GGA GCC AAC TTC CAA CCA AAT GGT AAT GCG     240
   Asn Met Ser Ile Asn Tyr Gly Ala Asn Phe Gln Pro Asn Gly Asn Ala
   65                  70                  75                  80
   TAT TTA TGC GTC TAT GGT TGG ACT GTT GAC CCT CTT GTC GAA TAT TAT     288
   Tyr Leu Cys Val Tyr Gly Trp Thr Val Asp Pro Leu Val Glu Tyr Tyr
                   85                  90                  95
   ATT GTC GAC AGT TGG GGC AAC TGG CGT CCA CCA GGA GCA ACG CCT AAG     336
   Ile Val Asp Ser Trp Gly Asn Trp Arg Pro Pro Gly Ala Thr Pro Lys
               100                 105                 110
   GGG ACC ATC ACT GTT GAT GGA GGA ACA TAT GAT ATC TAC GAG ACT CTT     384
   Gly Thr Ile Thr Val Asp Gly Gly Thr Tyr Asp Ile Tyr Glu Thr Leu
           115                 120                 125
   AGA GTC AAT CAA CCC TCC ATT AAG GGG ATT GCC ACA TTT AAA CAA TAT     432
   Arg Val Asn Gln Pro Ser Ile Lys Gly Ile Ala Thr Phe Lys Gln Tyr
       130                 135                 140
   TGG AGT GTT CGA AGA TCG AAA CGC ACG AGT GGC ACG ATT TCT GTC AGC      480
   Trp Ser Val Arg Arg Ser Lys Arg Thr Ser Gly Thr Ile Ser Val Ser
   145                 150                 155                 160
   AAC CAC TTT AGA GCG TGG GAA AAC TTA GGG ATG AAT ATG GGG AAA ATG      528
   Asn His Phe Arg Ala Trp Glu Asn Leu Gly Met Asn Met Gly Lys Met
                   165                 170                 175
   TAT GAA GTC GCG CTT ACT GTA GAA GGC TAT CAA AGT AGC GGA AGT GCT      576
   Tyr Glu Val Ala Leu Thr Val Glu Gly Tyr Gln Ser Ser Gly Ser Ala
               180                 185                 190
   AAT GTA TAT AGC AAT ACA CTA AGA ATT AAC GGT AAC CCT CTC TCA ACT      624
   Asn Val Tyr Ser Asn Thr Leu Arg Ile Asn Gly Asn Pro Leu Ser Thr
           195                 200                 205
   ATT AGT AAT GAC GAG AGC ATA ACT TTG GAT AAA AAC AAT                  663
   Ile Ser Asn Asp Glu Ser Ile Thr Leu Asp Lys Asn Asn
       210                 215                 220
(2) INFORMATION POUR LA SEQ ID NO:3:
   (i) CARACTERISTIQUES DE LA SEQUENCE :
      (A) LONGUEUR : 221 acides aminés
      (B) TYPE : acide aminé
      (D) CONFIGURATION : linéaire
   (ii) TYPE DE MOLECULE : protéine
   (v) TYPE DE FRAGMENT : fragment interne
   (vi) ORIGINE :
      (A) ORGANISME : Bacillus
      (B) SOUCHE : 720/1
   (xi) DESCRIPTION DE LA SEQUENCE: SEQ ID NO:3:
   Gln Ile Val Thr Asp Asn Ser Ile Gly Asn His Asp Gly Tyr Asp Tyr
   1 5 10 15
   Glu Phe Trp Lys Asp Ser Gly Gly Ser Gly Thr Met Ile Leu Asn His
   20 25 30
   Gly Gly Thr Phe Ser Ala Gln Trp Asn Asn Val Asn Asn Ile Leu Phe
   35 40 45
   Arg Lys Gly Lys Lys Phe Asn Glu Thr Gln Thr His Gln Gln Val Gly
   50 55 60
   Asn Met Ser Ile Asn Tyr Gly Ala Asn Phe Gln Pro Asn Gly Asn Ala
   65 70 75 80
   Tyr Leu Cys Val Tyr Gly Trp Thr Val Asp Pro Leu Val Glu Tyr Tyr
   85 90 95
   Ile Val Asp Ser Trp Gly Asn Trp Arg Pro Pro Gly Ala Thr Pro Lys
   100 105 110
   Gly Thr Ile Thr Val Asp Gly Gly Thr Tyr Asp Ile Tyr Glu Thr Leu
   115 120 125
   Arg Val Asn Gln Pro Ser Ile Lys Gly Ile Ala Thr Phe Lys Gln Tyr
   130 135 140
   Trp Ser Val Arg Arg Ser Lys Arg Thr Ser Gly Thr Ile Ser Val Ser
   145 150 155 160
   Asn His Phe Arg Ala Trp Glu Asn Leu Gly Met Asn Met Gly Lys Met
   165 170 175
   Tyr Glu Val Ala Leu Thr Val Glu Gly Tyr Gln Ser Ser Gly Ser Ala
   180 185 190
   Asn Val Tyr Ser Asn Thr Leu Arg Ile Asn Gly Asn Pro Leu Ser Thr
           195                 200                 205
   Ile Ser Asn Asp Glu Ser Ile Thr Leu Asp Lys Asn Asn
       210                 215                 220
(2) INFORMATION POUR LA SEQ ID NO:4:
   (i) CARACTERISTIQUES DE LA SEQUENCE :
      (A) LONGUEUR : 744 paires de bases
      (B) TYPE : acide nucléique
      (C) NOMBRE DE BRINS : simple
      (D) CONFIGURATION : linéaire
   (ii) TYPE DE MOLECULE : ADN génomique
   (xi) DESCRIPTION DE LA SEQUENCE: SEQ ID NO:4:
   ATGAGACAAA AGAAATTGAC GTTGATTTTA GCCTTTTTAG TTTGTTTTGC ACTAACCTTA     60
   CCTGCAGAAA TAATTCAGGC ACAAATCGTC ACCGACAATT CCATTGGCAA CCACGATGGC    120
   TATGATTATG AATTTTGGAA AGATAGCGGT GGCTCTGGGA CAATGATTCT CAATCATGGC    180
   GGTACGTTCA GTGCCCAATG GAACAATGTT AACAACATAT TATTCCGTAA AGGTAAAAAA    240
   TTCAATGAAA CACAAACACA CCAACAAGTT GGTAACATGT CCATAAACTA CGGAGCCAAC    300
   TTCCAACCAA ATGGTAATGC GTATTTATGC GTCTATGGTT GGACTGTTGA CCCTCTTGTC    360
   GAATATTATA TTGTCGACAG TTGGGGCAAC TGGCGTCCAC CAGGAGCAAC GCCTAAGGGG    420
   ACCATCACTG TTGATGGAGG AACATATGAT ATCTACGAGA CTCTTAGAGT CAATCAACCC    480
   TCCATTAAGG GGATTGCCAC ATTTAAACAA TATTGGAGTG TTCGAAGATC GAAACGCACG    540
   AGTGGCACGA TTTCTGTCAG CAACCACTTT AGAGCGTGGG AAAACTTAGG GATGAATATG     600
   GGGAAAATGT ATGAAGTCGC GCTTACTGTA GAAGGCTATC AAAGTAGCGG AAGTGCTAAT     660
   GTATATAGCA ATACACTAAG AATTAACGGT AACCCTCTCT CAACTATTAG TAATGACGAG     720
   AGCATAACTT TGGATAAAAA CAAT                                            744
(2) INFORMATION POUR LA SEQ ID NO:5:
   (i) CARACTERISTIQUES DE LA SEQUENCE :
      (A) LONGUEUR : 744 paires de bases
      (B) TYPE : acide nucléique
      (C) NOMBRE DE BRINS : simple
      (D) CONFIGURATION : linéaire
   (ii) TYPE DE MOLECULE : ADN génomique
   (xi) DESCRIPTION DE LA SEQUENCE: SEQ ID NO:5:
(2) INFORMATION POUR LA SEQ ID NO:6:
   (i) CARACTERISTIQUES DE LA SEQUENCE :
      (A) LONGUEUR : 248 acides aminés
      (B) TYPE : acide aminé
      (D) CONFIGURATION : linéaire
   (ii) TYPE DE MOLECULE : protéine
   (v) TYPE DE FRAGMENT : fragment interne
   (vi) ORIGINE :
      (A) ORGANISME : Bacillus
      (B) SOUCHE : 720/1
   (xi) DESCRIPTION DE LA SEQUENCE: SEQ ID NO:6:
   Met Arg Gln Lys Lys Leu Thr Leu Ile Leu Ala Phe Leu Val Cys Phe
           -25                 -20                 -15
   Ala Leu Thr Leu Pro Ala Glu Ile Ile Gln Ala Gln Ile Val Thr Asp
       -10                     -5              -1   1               5
   Asn Ser Ile Gly Asn His Asp Gly Tyr Asp Tyr Glu Phe Trp Lys Asp
   10 15 20
   Ser Gly Gly Ser Gly Thr Met Ile Leu Asn His Gly Gly Thr Phe Ser
   25 30 35
   Ala Gln Trp Asn Asn Val Asn Asn Ile Leu Phe Arg Lys Gly Lys Lys
   40 45 50
   Phe Asn Glu Thr Gln Thr His Gln Gln Val Gly Asn Met Ser Ile Asn
   55 60 65
   Tyr Gly Ala Asn Phe Gln Pro Asn Gly Asn Ala Tyr Leu Cys Val Tyr
   70 75 80 85
   Gly Trp Thr Val Asp Pro Leu Val Glu Tyr Tyr Ile Val Asp Ser Trp
   90 95 100
   Gly Asn Trp Arg Pro Pro Gly Ala Thr Pro Lys Gly Thr Ile Thr Val
   105 110 115
   Asp Gly Gly Thr Tyr Asp Ile Tyr Glu Thr Leu Arg Val Asn Gln Pro
   120 125 130
   Ser Ile Lys Gly Ile Ala Thr Phe Lys Gln Tyr Trp Ser Val Arg Arg
   135 140 145
   Ser Lys Arg Thr Ser Gly Thr Ile Ser Val Ser Asn His Phe Arg Ala
   150 155 160 165
   Trp Glu Asn Leu Gly Met Asn Met Gly Lys Met Tyr Glu Val Ala Leu
   170 175 180
   Thr Val Glu Gly Tyr Gln Ser Ser Gly Ser Ala Asn Val Tyr Ser Asn
   185 190 195
   Thr Leu Arg Ile Asn Gly Asn Pro Leu Ser Thr Ile Ser Asn Asp Glu
           200                 205                 210
   Ser Ile Thr Leu Asp Lys Asn Asn
       215                 220
(2) INFORMATION POUR LA SEQ ID NO:7:
   (i) CARACTERISTIQUES DE LA SEQUENCE :
      (A) LONGUEUR : 81 paires de bases
      (B) TYPE : acide nucléique
      (C) NOMBRE DE BRINS : simple
      (D) CONFIGURATION : linéaire
   (ii) TYPE DE MOLECULE : ADN génomique
   (xi) DESCRIPTION DE LA SEQUENCE: SEQ ID NO:7:
   ATGAGACAAA AGAAATTGAC GTTGATTTTA GCCTTTTTAG TTTGTTTTGC ACTAACCTTA         60
   CCTGCAGAAA TAATTCAGGC A                                                   81
(2) INFORMATION POUR LA SEQ ID NO:8:
   (i) CARACTERISTIQUES DE LA SEQUENCE :
      (A) LONGUEUR : 81 paires de bases
      (B) TYPE : acide nucléique
      (C) NOMBRE DE BRINS : simple
      (D) CONFIGURATION : linéaire
   (ii) TYPE DE MOLECULE : ADN génomique
   (xi) DESCRIPTION DE LA SEQUENCE: SEQ ID NO:8:
   ATG AGA CAA AAG AAA TTG ACG TTG ATT TTA GCC TTT TTA GTT TGT TTT         48
   Met Arg Gln Lys Lys Leu Thr Leu Ile Leu Ala Phe Leu Val Cys Phe
     1               5                  10                  15
   GCA CTA ACC TTA CCT GCA GAA ATA ATT CAG GCA                             81
   Ala Leu Thr Leu Pro Ala Glu Ile Ile Gln Ala
               20                  25
(2) INFORMATION POUR LA SEQ ID NO:9:
   (i) CARACTERISTIQUES DE LA SEQUENCE :
      (A) LONGUEUR : 27 acides aminés
      (B) TYPE : acide aminé
      (D) CONFIGURATION : linéaire
   (ii) TYPE DE MOLECULE : peptide
   (v) TYPE DE FRAGMENT : fragment interne
   (vi) ORIGINE :
      (A) ORGANISME : Bacillus
      (B) SOUCHE : 720/1
   (xi) DESCRIPTION DE LA SEQUENCE: SEQ ID NO:9:
   Met Arg Gln Lys Lys Leu Thr Leu Ile Leu Ala Phe Leu Val Cys Phe
    1               5                  10                  15
   Ala Leu Thr Leu Pro Ala Glu Ile Ile Gln Ala
               20                  25
(2) INFORMATION POUR LA SEQ ID NO:10:
   (i) CARACTERISTIQUES DE LA SEQUENCE :
      (A) LONGUEUR : 1513 paires de bases
      (B) TYPE : acide nucléique
      (C) NOMBRE DE BRINS : simple
      (D) CONFIGURATION : linéaire
   (ii) TYPE DE MOLECULE : ADN génomique
   (xi) DESCRIPTION DE LA SEQUENCE: SEQ ID NO:10:
   AAATTGAATT GTGTATATCT AATGATAACG ACAAATCGTC ACTGTTTTTA AACTAATCTC 60
   AAACCAATAC TTCTTTATTT AACGCTAACC ACTTGCAATC TTATCACAAG AACATTCTTT 120
   ATAGGAACTT TCCCATTTGC AAGACGATAA AAAATCTTTT TCCCCTATTT TATCTTATCG 180
   CCTTGATCGG TTTAATTTGT AAACTTTATT TTAGTTTACG TGATGTTCCC TCATTCATAC 240
   CATTAATCAC AGTTAACGCT AGAGTCATCT TTTTTCGGTT CTCAAAAATA CCTGAAGAAC 300
   ATTTATGTCA TATTTTCTCA CGCCGCTCCA TAATGGAATA TATATACTCT TTTATACATA 360
   TTAAGTAAAT TAGTATATAC TTGCGTTATC AAAATGTGAG ATAATCTAAT TGATCAAACA 420
   AGCAGCTATC CAAAAAACAC TGATGTTGAC CTCTTAAAGA AGTGTCACTA TCTATGAAAA 480
   GATAATTATC CAGTTTCAAA ATTTGAAATA GTGTGTATGG AATAGTTTGA ATGTCAACTG 540
   CTGTGAAAGG AGGGTAGGTA GTACCGTAGA CTTCATTACC AAAAATTAGT TGTAAAAAAA 600
   TTAAAAGGAG GAATGCCTAA TGAGACAAAA GAAATTGACG TTGATTTTAG CCTTTTTAGT 660
   TTGTTTTGCA CTAACCTTAC CTGCAGAAAT AATTCAGGCA CAAATCGTCA CCGACAATTC 720
   CATTGGCAAC CACGATGGCT ATGATTATGA ATTTTGGAAA GATAGCGGTG GCTCTGGGAC 780
   AATGATTCTC AATCATGGCG GTACGTTCAG TGCCCAATGG AACAATGTTA ACAACATATT 840
   ATTCCGTAAA GGTAAAAAAT TCAATGAAAC ACAAACACAC CAACAAGTTG GTAACATGTC 900
   CATAAACTAC GGAGCCAACT TCCAACCAAA TGGTAATGCG TATTTATGCG TCTATGGTTG 960
   GACTGTTGAC CCTCTTGTCG AATATTATAT TGTCGACAGT TGGGGCAACT GGCGTCCACC 1020
   AGGAGCAACG CCTAAGGGGA CCATCACTGT TGATGGAGGA ACATATGATA TCTACGAGAC 1080
   TCTTAGAGTC AATCAACCCT CCATTAAGGG GATTGCCACA TTTAAACAAT ATTGGAGTGT 1140
   TCGAAGATCG AAACGCACGA GTGGCACGAT TTCTGTCAGC AACCACTTTA GAGCGTGGGA 1200
   AAACTTAGGG ATGAATATGG GGAAAATGTA TGAAGTCGCG CTTACTGTAG AAGGCTATCA 1260
   AAGTAGCGGA AGTGCTAATG TATATAGCAA TACACTAAGA ATTAACGGTA ACCCTCTCTC 1320
   AACTATTAGT AATGACGAGA GCATAACTTT GGATAAAAAC AATTAAAAAT CCTTATCTCT 1380
   TTCGGTTCAG TTCTCATTAT TTTCAAATAA CCTCCCGGTT GGATCTTTTC CAACGGGAGG 1440
   TTTTATTGGA AAGGTTAAGT ATAGTATACT CCGATTCCAT CCAGAGGAAT GCTTGAAACA 1500
   CCTCCGTCAC TAG 1513
(2) INFORMATION POUR LA SEQ ID NO:11:
   (i) CARACTERISTIQUES DE LA SEQUENCE :
      (A) LONGUEUR : 1513 paires de bases
      (B) TYPE : acide nucléique
      (C) NOMBRE DE BRINS : simple
      (D) CONFIGURATION : linéaire
   (ii) TYPE DE MOLECULE : ADN génomique
   (xi) DESCRIPTION DE LA SEQUENCE: SEQ ID NO:11:
   AAATTGAATT GTGTATATCT AATGATAACG ACAAATCGTC ACTGTTTTTA AACTAATCTC 60
   AAACCAATAC TTCTTTATTT AACGCTAACC ACTTGCAATC TTATCACAAG AACATTCTTT        120
   ATAGGAACTT TCCCATTTGC AAGACGATAA AAAATCTTTT TCCCCTATTT TATCTTATCG        180
   CCTTGATCGG TTTAATTTGT AAACTTTATT TTAGTTTACG TGATGTTCCC TCATTCATAC        240
   CATTAATCAC AGTTAACGCT AGAGTCATCT TTTTTCGGTT CTCAAAAATA CCTGAAGAAC        300
   ATTTATGTCA TATTTTCTCA CGCCGCTCCA TAATGGAATA TATATACTCT TTTATACATA        360
   TTAAGTAAAT TAGTATATAC TTGCGTTATC AAAATGTGAG ATAATCTAAT TGATCAAACA        420
   AGCAGCTATC CAAAAAACAC TGATGTTGAC CTCTTAAAGA AGTGTCACTA TCTATGAAAA        480
   GATAATTATC CAGTTTCAAA ATTTGAAATA GTGTGTATGG AATAGTTTGA ATGTCAACTG        540
   CTGTGAAAGG AGGGTAGGTA GTACCGTAGA CTTCATTACC AAAAATTAGT TGTAAAAAAA        600
   TTAAAAGGAG GAATGCCTA  ATG AGA CAA AAG AAA TTG ACG TTG ATT TTA GCC        652
                         Met Arg Gln Lys Lys Leu Thr Leu Ile Leu Ala
                                     -25                 -20
   TTT TTA GTT TGT TTT GCA CTA ACC TTA CCT GCA GAA ATA ATT CAG GCA          700
   Phe Leu Val Cys Phe Ala Leu Thr Leu Pro Ala Glu Ile Ile Gln Ala
       -15                -10                 -5              -1
   CAA ATC GTC ACC GAC AAT TCC ATT GGC AAC CAC GAT GGC TAT GAT TAT          748
   Gln Ile Val Thr Asp Asn Ser Ile Gly Asn His Asp Gly Tyr Asp Tyr
     1               5                  10                  15
   GAA TTT TGG AAA GAT AGC GGT GGC TCT GGG ACA ATG ATT CTC AAT CAT          796
   Glu Phe Trp Lys Asp Ser Gly Gly Ser Gly Thr Met Ile Leu Asn His
               20                 25                  30
   GGC GGT ACG TTC AGT GCC CAA TGG AAC AAT GTT AAC AAC ATA TTA TTC        844
   Gly Gly Thr Phe Ser Ala Gln Trp Asn Asn Val Asn Asn Ile Leu Phe
           35                  40                  45
   CGT AAA GGT AAA AAA TTC AAT GAA ACA CAA ACA CAC CAA CAA GTT GGT        892
   Arg Lys Gly Lys Lys Phe Asn Glu Thr Gln Thr His Gln Gln Val Gly
       50                  55                  60
   AAC ATG TCC ATA AAC TAC GGA GCC AAC TTC CAA CCA AAT GGT AAT GCG        940
   Asn Met Ser Ile Asn Tyr Gly Ala Asn Phe Gln Pro Asn Gly Asn Ala
   65                  70                  75                  80
   TAT TTA TGC GTC TAT GGT TGG ACT GTT GAC CCT CTT GTC GAA TAT TAT        988
   Tyr Leu Cys Val Tyr Gly Trp Thr Val Asp Pro Leu Val Glu Tyr Tyr
                   85                  90                  95
   ATT GTC GAC AGT TGG GGC AAC TGG CGT CCA CCA GGA GCA ACG CCT AAG       1036
   Ile Val Asp Ser Trp Gly Asn Trp Arg Pro Pro Gly Ala Thr Pro Lys
               100                 105                 110
   GGG ACC ATC ACT GTT GAT GGA GGA ACA TAT GAT ATC TAC GAG ACT CTT       1084
   Gly Thr Ile Thr Val Asp Gly Gly Thr Tyr Asp Ile Tyr Glu Thr Leu
           115                 120                 125
   AGA GTC AAT CAA CCC TCC ATT AAG GGG ATT GCC ACA TTT AAA CAA TAT       1132
   Arg Val Asn Gln Pro Ser Ile Lys Gly Ile Ala Thr Phe Lys Gln Tyr
       130                 135                 140
   TGG AGT GTT CGA AGA TCG AAA CGC ACG AGT GGC ACG ATT TCT GTC AGC       1180
   Trp Ser Val Arg Arg Ser Lys Arg Thr Ser Gly Thr Ile Ser Val Ser
   145                 150                 155                 160
   AAC CAC TTT AGA GCG TGG GAA AAC TTA GGG ATG AAT ATG GGG AAA ATG       1228
   Asn His Phe Arg Ala Trp Glu Asn Leu Gly Met Asn Met Gly Lys Met
                   165                 170                 175
   TAT GAA GTC GCG CTT ACT GTA GAA GGC TAT CAA AGT AGC GGA AGT GCT          1276
   Tyr Glu Val Ala Leu Thr Val Glu Gly Tyr Gln Ser Ser Gly Ser Ala
               180                 185                 190
   AAT GTA TAT AGC AAT ACA CTA AGA ATT AAC GGT AAC CCT CTC TCA ACT          1324
   Asn Val Tyr Ser Asn Thr Leu Arg Ile Asn Gly Asn Pro Leu Ser Thr
           195                 200                 205
   ATT AGT AAT GAC GAG AGC ATA ACT TTG GAT AAA AAC AAT TAAAAATCCT           1373
   Ile Ser Asn Asp Glu Ser Ile Thr Leu Asp Lys Asn Asn
       210                 215                 220
   TATCTCTTTC GGTTCAGTTC TCATTATTTT CAAATAACCT CCCGGTTGGA TCTTTTCCAA        1433
   CGGGAGGTTT TATTGGAAAG GTTAAGTATA GTATACTCCG ATTCCATCCA GAGGAATGCT        1493
   TGAAACACCT CCGTCACTAG                                                    1513
(2) INFORMATION POUR LA SEQ ID NO:12:
   (i) CARACTERISTIQUES DE LA SEQUENCE :
      (A) LONGUEUR : 619 paires de bases
      (B) TYPE : acide nucléique
      (C) NOMBRE DE BRINS : simple
      (D) CONFIGURATION : linéaire
   (ii) TYPE DE MOLECULE : ADN génomique
   (xi) DESCRIPTION DE LA SEQUENCE: SEQ ID NO:12:
   AAATTGAATT GTGTATATCT AATGATAACG ACAAATCGTC ACTGTTTTTA AACTAATCTC          60
   AAACCAATAC TTCTTTATTT AACGCTAACC ACTTGCAATC TTATCACAAG AACATTCTTT         120
   ATAGGAACTT TCCCATTTGC AAGACGATAA AAAATCTTTT TCCCCTATTT TATCTTATCG         180
   CCTTGATCGG TTTAATTTGT AAACTTTATT TTAGTTTACG TGATGTTCCC TCATTCATAC 240
   CATTAATCAC AGTTAACGCT AGAGTCATCT TTTTTCGGTT CTCAAAAATA CCTGAAGAAC 300
   ATTTATGTCA TATTTTCTCA CGCCGCTCCA TAATGGAATA TATATACTCT TTTATACATA 360
   TTAAGTAAAT TAGTATATAC TTGCGTTATC AAAATGTGAG ATAATCTAAT TGATCAAACA 420
   AGCAGCTATC CAAAAAACAC TGATGTTGAC CTCTTAAAGA AGTGTCACTA TCTATGAAAA 480
   GATAATTATC CAGTTTCAAA ATTTGAAATA GTGTGTATGG AATAGTTTGA ATGTCAACTG 540
   CTGTGAAAGG AGGGTAGGTA GTACCGTAGA CTTCATTACC AAAAATTAGT TGTAAAAAAA 600
   TTAAAAGGAG GAATGCCTA 619
(2) INFORMATION POUR LA SEQ ID NO:13:
   (i) CARACTERISTIQUES DE LA SEQUENCE :
      (A) LONGUEUR : 150 paires de bases
      (B) TYPE : acide nucléique
      (C) NOMBRE DE BRINS : simple
      (D) CONFIGURATION : linéaire
   (ii) TYPE DE MOLECULE : ADN génomique
   (xi) DESCRIPTION DE LA SEQUENCE: SEQ ID NO:13:
   TAAAAATCCT TATCTCTTTC GGTTCAGTTC TCATTATTTT CAAATAACCT CCCGGTTGGA 60
   TCTTTTCCAA CGGGAGGTTT TATTGGAAAG GTTAAGTATA GTATACTCCG ATTCCATCCA 120
   GAGGAATGCT TGAAACACCT CCGTCACTAG 150
(2) INFORMATION POUR LA SEQ ID NO:14:
   (i) CARACTERISTIQUES DE LA SEQUENCE :
      (A) LONGUEUR : 56 paires de bases
      (B) TYPE : acide nucléique
      (C) NOMBRE DE BRINS : simple
      (D) CONFIGURATION : linéaire
   (ii) TYPE DE MOLECULE : acide nucléique (oligonucléotide synthétique)
   (xi) DESCRIPTION DE LA SEQUENCE: SEQ ID NO:14:
   CCCCCCTACG TAGCGGCCGC CCCGGCCGGT AACCTAGGAA GTCAGCGCCC TGCACC 56
(2) INFORMATION POUR LA SEQ ID NO:15:
   (i) CARACTERISTIQUES DE LA SEQUENCE :
      (A) LONGUEUR : 56 paires de bases
      (B) TYPE : acide nucléique
      (C) NOMBRE DE BRINS : simple
      (D) CONFIGURATION : linéaire
   (ii) TYPE DE MOLECULE : acide nucléique (oligonucléotide synthétique)
   (xi) DESCRIPTION DE LA SEQUENCE: SEQ ID NO:15:
   CCCCCCTACG TAGGCCGGGG CGGCCGCGGT TACCTAGGGC CTCGTGATAC GCCTAT 56
(2) INFORMATION POUR LA SEQ ID NO:16:
   (i) CARACTERISTIQUES DE LA SEQUENCE :
      (A) LONGUEUR : 31 paires de bases
      (B) TYPE : acide nucléique
      (C) NOMBRE DE BRINS : simple
      (D) CONFIGURATION : linéaire
   (ii) TYPE DE MOLECULE : acide nucléique (oligonucléotide synthétique)
   (xi) DESCRIPTION DE LA SEQUENCE: SEQ ID NO:16:
   ACGAGGAAAG ATGCTGTTCT TGTAAATGAG T 31
(2) INFORMATION POUR LA SEQ ID NO:17:
   (i) CARACTERISTIQUES DE LA SEQUENCE :
      (A) LONGUEUR : 19 paires de bases
      (B) TYPE : acide nucléique
      (C) NOMBRE DE BRINS : simple
      (D) CONFIGURATION : linéaire
   (ii) TYPE DE MOLECULE : acide nucléique (oligonucléotide synthétique)
   (xi) DESCRIPTION DE LA SEQUENCE: SEQ ID NO:17:
   TACCTTGTCT ACAAACCCC 19
(2) INFORMATION POUR LA SEQ ID NO:18:
   (i) CARACTERISTIQUES DE LA SEQUENCE :
      (A) LONGUEUR : 18 paires de bases
      (B) TYPE : acide nucléique
      (C) NOMBRE DE BRINS : simple
      (D) CONFIGURATION : linéaire
   (ii) TYPE DE MOLECULE : acide nucléique (oligonucléotide synthétique)
   (xi) DESCRIPTION DE LA SEQUENCE: SEQ ID NO:18:
   CGGTCGCCGC ATACACTA                                                    18
(2) INFORMATION POUR LA SEQ ID NO:19:
   (i) CARACTERISTIQUES DE LA SEQUENCE :
      (A) LONGUEUR : 36 paires de bases
      (B) TYPE : acide nucléique
      (C) NOMBRE DE BRINS : simple
      (D) CONFIGURATION : linéaire
   (ii) TYPE DE MOLECULE : acide nucléique (oligonucléotide synthétique)
   (xi) DESCRIPTION DE LA SEQUENCE: SEQ ID NO:19:
   CCCCCCCCCG GTAACCTGCA TTAATGAATC GGCCAA                                36
(2) INFORMATION POUR LA SEQ ID NO:20:
   (i) CARACTERISTIQUES DE LA SEQUENCE :
      (A) LONGUEUR : 39 paires de bases
      (B) TYPE : acide nucléique
      (C) NOMBRE DE BRINS : simple
      (D) CONFIGURATION : linéaire
   (ii) TYPE DE MOLECULE : acide nucléique (oligonucléotide synthétique)
   (xi) DESCRIPTION DE LA SEQUENCE: SEQ ID NO:20:
   CCCCCCCCCG GTTACCGTAT TTATTAACTT CTCCTAGTA                             39
(2) INFORMATION POUR LA SEQ ID NO:21:
   (i) CARACTERISTIQUES DE LA SEQUENCE :
      (A) LONGUEUR : 50 paires de bases
      (B) TYPE : acide nucléique
      (C) NOMBRE DE BRINS : simple
      (D) CONFIGURATION : linéaire
   (ii) TYPE DE MOLECULE : acide nucléique (oligonucléotide synthétique)
   (xi) DESCRIPTION DE LA SEQUENCE: SEQ ID NO:21:
   CCCCCCTCTA GATTAATTAA CCAAGCTTGG GATCCGTCGA CCTGCAGATC 50
(2) INFORMATION POUR LA SEQ ID NO:22:
   (i) CARACTERISTIQUES DE LA SEQUENCE :
      (A) LONGUEUR : 39 paires de bases
      (B) TYPE : acide nucléique
      (C) NOMBRE DE BRINS : simple
      (D) CONFIGURATION : linéaire
   (ii) TYPE DE MOLECULE : acide nucléique (oligonucléotide synthétique)
   (xi) DESCRIPTION DE LA SEQUENCE: SEQ ID NO:22:
   CCCCCCTGAA ATCAGCTGGA CTAAAAGGGA TGCAATTTC 39
(2) INFORMATION POUR LA SEQ ID NO:23:
   (i) CARACTERISTIQUES DE LA SEQUENCE :
      (A) LONGUEUR : 30 paires de bases
      (B) TYPE : acide nucléique
      (C) NOMBRE DE BRINS : simple
      (D) CONFIGURATION : linéaire
   (ii) TYPE DE MOLECULE : acide nucléique (oligonucléotide synthétique)
   (xi) DESCRIPTION DE LA SEQUENCE: SEQ ID NO:23:
   CCCCCCGTCG ACCGCATGCG CCGGCACAGC 30
(2) INFORMATION POUR LA SEQ ID NO:24:
   (i) CARACTERISTIQUES DE LA SEQUENCE :
      (A) LONGUEUR : 39 paires de bases
      (B) TYPE : acide nucléique
      (C) NOMBRE DE BRINS : simple
      (D) CONFIGURATION : linéaire
   (ii) TYPE DE MOLECULE : acide nucléique (oligonucléotide synthétique)
   (xi) DESCRIPTION DE LA SEQUENCE: SEQ ID NO:24:
   CCCCCCGCAT GCGCAAATCG TCACCGACAA TTCCATTGG 39
(2) INFORMATION POUR LA SEQ ID NO:25:
   (i) CARACTERISTIQUES DE LA SEQUENCE :
      (A) LONGUEUR : 19 paires de bases
      (B) TYPE : acide nucléique
      (C) NOMBRE DE BRINS : simple
      (D) CONFIGURATION : linéaire
   (ii) TYPE DE MOLECULE : acide nucléique (oligonucléotide synthétique)
   (xi) DESCRIPTION DE LA SEQUENCE: SEQ ID NO:25:
   TACCTTGTCT ACAAACCCC                                              19
(2) INFORMATION POUR LA SEQ ID NO:26:
   (i) CARACTERISTIQUES DE LA SEQUENCE :
      (A) LONGUEUR : 185 paires de bases
      (B) TYPE : acide nucléique
      (C) NOMBRE DE BRINS : simple
      (D) CONFIGURATION : linéaire
   (ii) TYPE DE MOLECULE : ADN génomique
   (xi) DESCRIPTION DE LA SEQUENCE: SEQ ID NO:26:
   TCATGTAACT CGCCTTGATC TATTTCATTT GTATCAAAGG ATTTATACAC AAACAAGAGA    60
   CATCCATGCC GGGTTAAAGC AGTATCGTTC CATCTAACAG AGAAGGNCTG CATGAAAGGA   120
   GGTGATGGGT TTTTCATCTT AGGGATGACA GAACAATACG GATGAAAAAA GGAGAGGGAT   180
   GGAAA
(2) INFORMATION POUR LA SEQ ID NO:27:
   (i) CARACTERISTIQUES DE LA SEQUENCE :
      (A) LONGUEUR : 81 paires de bases
      (B) TYPE : acide nucléique
      (C) NOMBRE DE BRINS : simple
      (D) CONFIGURATION : linéaire
   (ii) TYPE DE MOLECULE : ADN génomique
   (xi) DESCRIPTION DE LA SEQUENCE: SEQ ID NO:27:
   ATGAATTTGA AAAGATTGAG GCTGTTGTTT GTGATGTGTA TTGGATTTGT GCTGACACTG    60
   ACGGCTGTGC CGGCTCATGC G                                              81
(2) INFORMATION POUR LA SEQ ID NO:28:
   (i) CARACTERISTIQUES DE LA SEQUENCE :
      (A) LONGUEUR : 81 paires de bases
      (B) TYPE : acide nucléique
      (C) NOMBRE DE BRINS : simple
      (D) CONFIGURATION : linéaire
   (ii) TYPE DE MOLECULE : ADN génomique
   (xi) DESCRIPTION DE LA SEQUENCE: SEQ ID NO:28:
   ATG AAT TTG AAA AGA TTG AGG CTG TTG TTT GTG ATG TGT ATT GGA TTT          48
   Met Asn Leu Lys Arg Leu Arg Leu Leu Phe Val Met Cys Ile Gly Phe
    1               5                  10                  15
   GTG CTG ACA CTG ACG GCT GTG CCG GCT CAT GCG                              81
   Val Leu Thr Leu Thr Ala Val Pro Ala His Ala
               20                  25
(2) INFORMATION POUR LA SEQ ID NO:29:
   (i) CARACTERISTIQUES DE LA SEQUENCE :
      (A) LONGUEUR : 27 acides aminés
      (B) TYPE : acide aminé
      (C) NOMBRE DE BRINS : simple
      (D) CONFIGURATION : linéaire
   (ii) TYPE DE MOLECULE : peptide
   (xi) DESCRIPTION DE LA SEQUENCE: SEQ ID NO:29:
   Met Asn Leu Lys Arg Leu Arg Leu Leu Phe Val Met Cys Ile Gly Phe
    1               5                  10                  15
   Val Leu Thr Leu Thr Ala Val Pro Ala His Ala
               20                  25

## Description

L'invention concerne une nouvelle xylanase. L'invention concerne également les procédés de préparation de cette xylanase, les utilisations de celle-ci et les compositions comprenant celle-ci.

L'invention concerne également une nouvelle souche de microorganismes produisant cette xylanase et une molécule d'ADN comprenant la séquence de nucléotides qui code pour cette xylanase. L'invention concerne également des vecteurs contenant cette molécule d'ADN et les souches transformées par ces vecteurs.

L'invention concerne également le promoteur dérivé du gène qui code pour la xylanase de Bacillus pumilus PRL B12 et la préséquence qui code pour le peptide signal de la xylanase de Bacillus pumilus PRL B12. L'invention concerne également des vecteurs, qui contiennent ce promoteur et cette préséquence, et également la molécule d'ADN comprenant la séquence de nucléotides qui code pour la partie mature de la xylanase de l'invention. L'invention concerne également des souches transformées par ces vecteurs.

Des xylanases thermostables, actives dans une large gamme de pH sont connues, telles que notamment des xylanases produites par des souches de bacille alcalophile, (Gupta, et al. BIOTECHNOLOGY LETTERS, 1992, 14 (11), pages 1045-1046 et demande de brevet international WO 94/04664). Cependant, malgré ces propriétés, ces enzymes sembleraient peu efficaces en blanchiment de pâte à papier.

En conséquence, il y a actuellement un besoin pour une xylanase utilisable lors du traitement de la pâte à papier, très stable et également très active dans une large gamme de température et de pH basique et acide.

Yu et al (1993) J Microbiol. Biotechnol 3,3: 139-145. Cet article concerne une xylanase sécrétée de l'espèce Bacille YA-14, et le peptide signalisant de là. Ces séquences sont comparées avec des autres séquences de la xylanase des espèces Bacille et Schizophyllum.

WO 95/18219 (Gist-Brocades) divulgue des xylanases qui tolèrent l'alcalinité et qui peuvent être obtenues des micro-organismes des bacilles gram-positifs qui tolèrent l'alcalinité.

EP 0 634 490 (Solvay) divulgue une xylanase purifiée du Bacille Pumilus PRL B12 et son usage dans B. licheniformis transformé.

La présente invention a pour but de fournir une nouvelle xylanase, active dans une large gamme de pH, aussi bien à pH alcalin qu'à pH acide.

La présente invention a également pour but d'identifier, isoler et fournir une souche, en particulier une souche de Bacillus, qui produit naturellement ladite xylanase.

La présente invention a également pour but d'isoler et de fournir une molécule d'ADN comprenant une séquence de nucléotides qui code pour ladite xylanase.

La présente invention a également pour but de préparer et fournir un vecteur d'expression contenant la séquence de nucléotides codant pour ladite xylanase.

La présente invention a également pour but de préparer et fournir un vecteur d'intégration contenant la séquence de nucléotides codant pour ladite xylanase.

La présente invention a également pour but de préparer et fournir le promoteur dérivé du gène qui code pour la xylanase de Bacillus pumilus PRL B12. La présente invention a également pour but de préparer et fournir la préséquence qui code pour le peptide signal de la xylanase de Bacillus pumilus PRL B12. Les vecteurs, qui comprennent ce promoteur et/ou cette préséquence, contiennent également la molécule d'ADN comprenant la séquence de nucléotides qui code pour la partie mature de la xylanase de l'invention. Les souches transformées par ces vecteurs produisent la xylanase de l'invention de façon hétérologue.

La présente invention a également pour but de préparer et fournir un hôte de Bacillus transformé avec le vecteur d'expression qui contient la molécule d'ADN comprenant la séquence de nucléotides de la souche de Bacillus codant pour ladite xylanase.

La présente invention a également pour but de préparer et fournir un hôte de Bacillus transformé avec le vecteur d'intégration qui contient la molécule d'ADN comprenant la séquence de nucléotides de la souche de Bacillus codant pour ladite xylanase.

La présente invention a également pour but de préparer et fournir une composition contenant cette xylanase.

La présente invention a également pour but de préparer et fournir une xylanase utilisable dans le traitement de la pâte à papier, et des pâtes ayant un pH basique, neutre ou acide, et notamment des pâtes ayant un pH particulièrement basique, et des pâtes à papier de diverses provenances, telles que les pâtes provenant d'arbres résineux, les pâtes provenant d'arbres feuillus, et particulièrement la pâte d'eucalyptus.

A cet effet, l'invention concerne une xylanase provenant d'un Bacillus, et plus particulièrement d'un microorganisme aérobie et non thermophile.

On utilise de préférence la souche de Bacillus sp. 720/1 ou un dérivé ou mutant de cette souche. La xylanase de l'invention est dérivée de (naturellement produite par) la souche de Bacillus sp. 720/1. La xylanase est classifiée dans le système international sous le numéro E.C. 3.2.1.8. Il s'agit d'une endo-1,4-bêta-xylanase.

De préférence la xylanase, isolée et purifiée, est constituée d'un seul type de polypeptide, ayant un poids moléculaire d'environ 25 kDa.

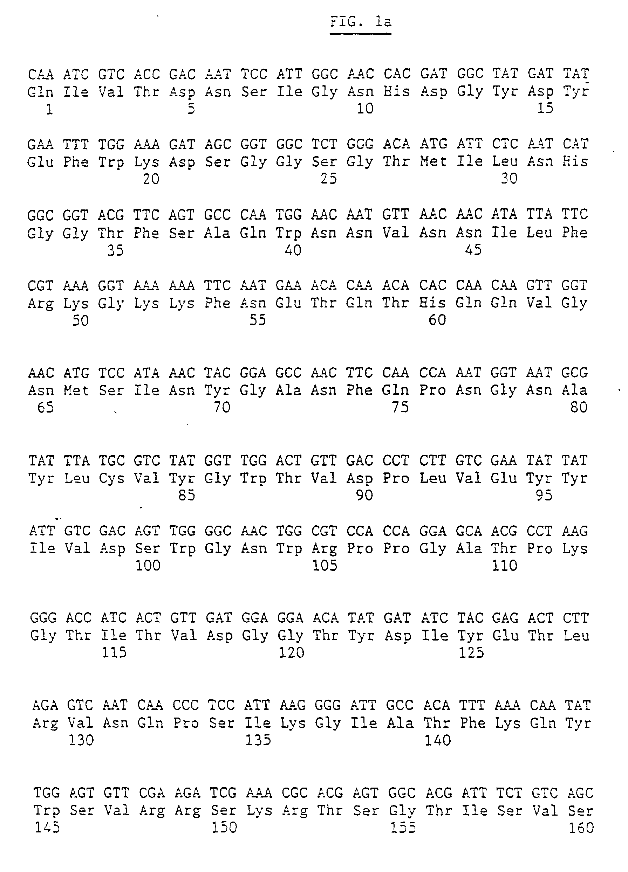

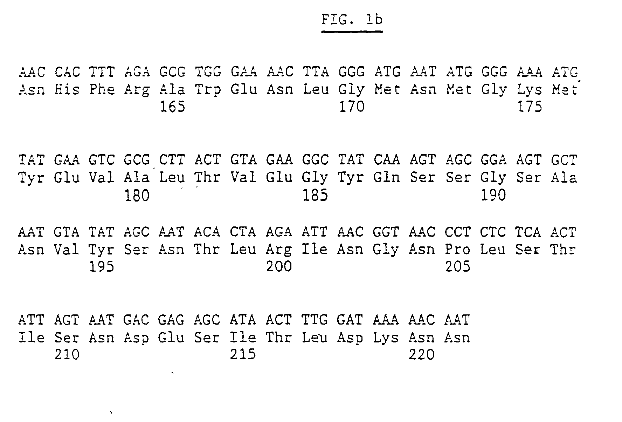

L'invention concerne une xylanase, isolée et purifiée, comprenant la séquence d'acides aminés de 1 à 221 acides aminés (SEQ ID NO:3) ou une séquence modifiée dérivée de celle-ci. La séquence d'acides aminés et la séquence de nucléotides (SEQ ID NO:1) codant pour la xylanase mature, ainsi que sa traduction en acides aminés (SEQ ID NO:2), est donnée à la figure 1 (figures 1a et 1b).

La xylanase de l'invention est synthétisée sous forme d'un précurseur. Le précurseur contient 248 acides aminés :(SEQ ID NO:6). On identifie la séquence de nucléotides (SEQ ID NO:4) codant pour le précurseur de la xylanase, ainsi que sa traduction en acides aminés (SEQ ID NO:5).

Le précurseur contient la séquence de 221 acides aminés (SEQ ID NO:3) de la xylanase mature et la séquence de 27 acides aminés (SEQ ID NO:9) de la préséquence.

La séquence de la xylanase mature est précédée d'une préséquence. Il s'agit d'une séquence additionnelle de 27 acides aminés (SEQ ID NO:9). On identifie la séquence de nucléotides correspondante (SEQ ID NO:7), ainsi que sa traduction en acides aminés (SEQ ID NO:8). Cette préséquence code pour le peptide signal de la xylanase de l'invention.

De manière particulièrement préférée, ladite xylanase a un point isoélectrique déterminé compris entre environ 9,5 et environ 9,7.

La xylanase selon l'invention est thermostable et active dans une large gamme de pH. De préférence, la xylanase selon l'invention est alcaline.

La xylanase selon l'invention possède par ailleurs toutes les propriétés adéquates compatibles avec les conditions industrielles réelles de traitement enzymatique de la pâte à papier. Selon les multiples étapes des divers traitements de la pâte à papier mis en oeuvre industriellement, ces propriétés sont une bonne stabilité vis-à-vis du pH et de la température, une activité enzymatique dans une large gamme de pH et de température, tels que notamment un pH compris entre environ 5 et 10 et une température comprise entre environ 50 et 80°C.

La xylanase de l'invention est active dans une gamme de pH supérieure ou égale à environ 5. La xylanase de l'invention est active dans une gamme de pH inférieure ou égale à environ 11. La xylanase développe une activité enzymatique de plus de 50 % de l'activité maximale, mesurée à une température d'environ 50 °C et en présence de xylane, dans une gamme de pH supérieure ou égale à environ 5,0. La xylanase développe une activité enzymatique de plus de 50 % de l'activité maximale, mesurée à une température d'environ 50°C et en présence de xylane, dans une gamme de pH inférieure à environ 10,5.

La xylanase de l'invention est active dans une gamme de température supérieure ou égale à environ 50°C. La xylanase de l'invention est active dans une gamme de température inférieure ou égale à environ 80°C. La xylanase développe une activité enzymatique de plus de 50 % de l'activité maximale, mesurée à un pH d'environ 9 et en présence de xylane, dans une gamme de température supérieure ou égale à environ 50°C. La xylanase développe une activité enzymatique de plus de 50 % de l'activité maximale, mesurée à un pH d'environ 9 et en présence de xylane, dans une gamme de température inférieure à environ 80°C.

L'invention permet l'obtention d'une xylanase modifiée, c'est-à-dire une enzyme dont la séquence en acides aminés diffère de celle de l'enzyme sauvage par au moins un acide aminé. Ces modifications peuvent être obtenues par des techniques classiques de mutagénèse sur l'ADN, telles que l'exposition à des rayonnements ultraviolets, à des produits chimiques, tels que l'éthyl méthane sulfonate (EMS), le N-méthyl-N-nitro-N-nitrosoguanidine (MNNG), le nitrite de sodium ou l'O-méthylhydroxylamine ou par des techniques de génie génétique, comme, par exemple, la mutagénèse dirigée ou la mutagénèse aléatoire. Ces techniques sont connues de l'homme du métier et sont notamment décrites dans MOLECULAR CLONING- a laboratory manual - SAMBROOK, FRITSCH, MANIATIS - second edition, 1989, chapitre 15.

L'invention permet l'obtention d'une xylanase ayant des propriétés immunochimiques identiques ou partiellement identiques à la xylanase obtenue à partir de la souche de Bacillus sp. 720/1. Les propriétés immunochimiques peuvent être déterminées de façon immunologique par des tests d'identité, notamment en utilisant des anticorps spécifiques, polyclonaux ou monoclonaux. Des tests d'identité sont connus de l'homme du métier, tels que notamment la méthode d'immunodiffusion d'Ouchterlony, ou la méthode d'immunoélectrophorèse. Des exemples de telles méthodes sont décrits par Axelsen N.H., Handbook of Immunoprecipitation Gel Techniques, Blackwell Scientific Publications, 1983, chapitres 5 et 14, les termes "identité antigénique" et "identité antigénique partielle" sont décrits dans ce document aux chapitres 5, 19 et 20. Un serum contenant l'anticorps specifique est préparé selon la méthode décrite en immunisant des animaux (par exemples souris, lapin ou chèvre) avec une préparation de xylanase purifiée. Cette préparation peut être mélangée avec un additif, tel que l'adjuvant de Freund, et le mélange obtenu est injecté à des animaux. L'anticorps polyclonal est obtenu après une ou plusieurs immunisations. Un exemple consiste à injecter, de façon sous cutanée à deux semaines d'intervalle, quatre fractions contenant chacune 150 microgrammes de xylanase purifiée, l'immunisation dure alors 8 semaines. Le sérum est prelevé après la période d'immunisation et l'immunoglobuline peut être isolée selon la méthode décrite par Axelsen N.H. (1983).

La présente invention concerne également l'identification et la fourniture d'une nouvelle bactérie, isolée et purifiée, aérobie produisant la xylanase. Ledit Bacillus est la souche de Bacillus sp. 720/1.

L'invention permet à l'homme du métier d'obtenir un dérivé ou mutant de cette souche. Par dérivé de cette souche, on entend toute bactérie modifiée naturellement. Les dérivés de cette souche peuvent être obtenus par les techniques connues de modification telles que culture sur milieu specifique, rayonnement ultraviolet, rayons X. Par mutant de cette souche, on entend toute bactérie modifiée artificiellement. Les mutants de cette souche peuvent être obtenus par les techniques connues de modification telles que l'exposition à des agents mutagènes, et les techniques de génie génétique. Ces techniques sont connues de l'homme du métier et sont notamment décrites dans SAMBROOK et al., 1989, chapitre 15.

La souche de Bacillus sp. 720/1 a été déposée à la collection nommée BELGIAN COORDINATED COLLECTIONS OF MICROORGANISMS (LMG culture collection, Université de Gand, Laboratoire de Microbiologie - K.L. Ledeganckstraat 35, B-9000 Gand, Belgique) conformément au Traité de Budapest sous le numéro LMG P-14798 le 9 juin 1994. L'invention concerne une culture isolée et purifiée de la souche de Bacillus sp. 720/1 et une culture dérivée ou mutée de celle-ci.

La souche de la présente invention a été identifiée par ses caractéristiques biochimiques : bactérie Gram positif, aérobie, qui se présente sous la forme de bâtonnet, elle forme une endospore. Elle est oligosporogène.

L'invention concerne également l'isolement et la fourniture d'une molécule d'ADN comprenant la séquence de nucléotides (SEQ ID NO:1) qui code pour la xylanase mature de Bacillus sp. 720/1 (LMG P-14798). De préférence cette molécule d'ADN comprend tout le gène de la xylanase de Bacillus sp. 720/1. Par tout le gène de la xylanase (SEQ ID NO:10), on entend au moins le (ou les) promoteur(s) de transcription, la (ou les) séquence(s) signal, la séquence de nucléotides codant pour la xylanase mature et le (ou les) terminateur(s) de transcription.

L'homme du métier peut obtenir une séquence modifiée, derivée de la molécule d'ADN codant pour la xylanase de l'invention.

Par séquence modifiée dérivée de la molécule d'ADN, on entend toute molécule d'ADN obtenue par modification d'un ou de plusieurs nucléotides du gène qui code pour la xylanase de l'invention. Les techniques d'obtention de telles séquences sont connues de l'homme du métier et sont notamment décrites dans MOLECULAR CLONING - a laboratory manual - SAMBROOK, FRITSCH, MANIATIS - second édition, 1989, chapitre 15. Habituellement, la séquence modifiée dérivée de la molécule d'ADN comprend au moins 70 % d'homologie avec la séquence de nucléotides (SEQ ID NO:1) du gène qui code pour la xylanase de l'invention, c'est-à-dire au moins 70 % de nucléotides identiques et ayant la même position dans la séquence. De préférence, la séquence modifiée dérivée de la molécule d'ADN comprend au moins 80 % d'homologie avec la séquence de nucléotides (SEQ ID NO:1) du gène qui code pour la xylanase de l'invention. De manière particulièrement préférée, la séquence modifiée dérivée de la molécule d'ADN comprend au moins 90 % d'homologie avec la séquence de nucléotides (SEQ ID NO:1) du gène qui code pour la xylanase.

La séquence complète de nucléotides codant pour la xylanase mature, ainsi que sa traduction en acides aminés (SEQ ID NO:2), est donnée à la figure 1 (figures 1a et 1b).

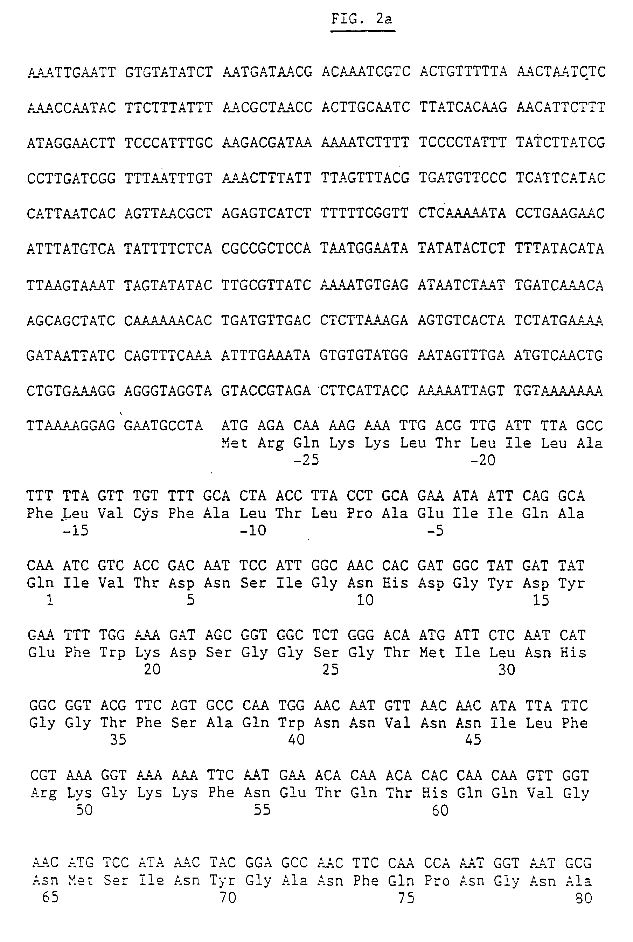

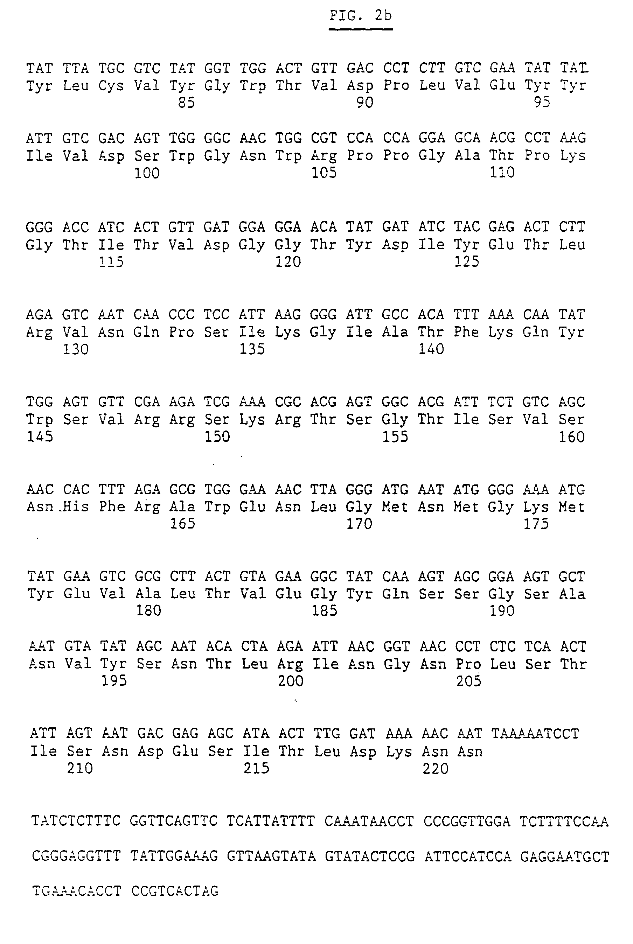

Habituellement, la molécule d'ADN, selon l'invention, comprend au moins la séquence de nucléotides (SEQ ID NO:4) qui code pour le précurseur de la xylanase. Cette séquence de nucléotides (SEQ ID NO:4) comprend la séquence de nucléotides (SEQ ID NO:1) codant pour la xylanase mature de Bacillus sp. 720/1 (LMG P-14798) et sa séquence signal (préséquence) (SEQ ID NO:7). De préférence, cette molécule d'ADN comprend tout le gène de la xylanase de Bacillus sp. 720/1 et, de manière particulièrement préférée, la séquence de nucléotides (SEQ ID NO:10). La séquence de nucléotides (SEQ ID NO:10) est constituée de, dans le sens amino-carboxy et de gauche à droite, la séquence de nucléotides (SEQ ID NO:12) qui comprend le promoteur de la xylanase, la séquence de nucléotides de la préséquence (SEQ ID NO:7), la séquence de nucléotides de la xylanase mature (SEQ ID NO:1) et la séquence de nucléotides (SEQ ID NO:13) qui comprend le terminateur de la xylanase. La figure 2 (figure 2a et figure 2b) représente la séquence de nucléotides du gène codant pour la xylanase ainsi que sa traduction en acides aminés (SEQ ID NO:11).

Dans une variante, l'invention concerne également une molécule d'ADN qui comprend le promoteur dérivé du gène qui code pour la xylanase de Bacillus pumilus PRL B12, une préséquence et la séquence de nucléotides (SEQ ID NO:1) qui code pour la xylanase de Bacillus sp. 720/1. Dans une autre variante, l'invention concerne également une molécule d'ADN qui comprend un promoteur, la préséquence qui code pour le peptide signal de la xylanase de Bacillus pumilus PRL B12 et la séquence de nucléotides (SEQ ID NO:1) qui code pour la xylanase de Bacillus sp. 720/1. De préférence, l'invention concerne une molécule d'ADN qui comprend le promoteur (SEQ ID NO:26) dérivé du gène qui code pour la xylanase de Bacillus pumilus PRL B12, la préséquence (SEQ ID NO:27) qui code pour le peptide signal de la xylanase de Bacillus pumilus PRL B12 et la séquence de nucléotides (SEQ ID NO:1) qui code pour la xylanase de Bacillus sp. 720/1.

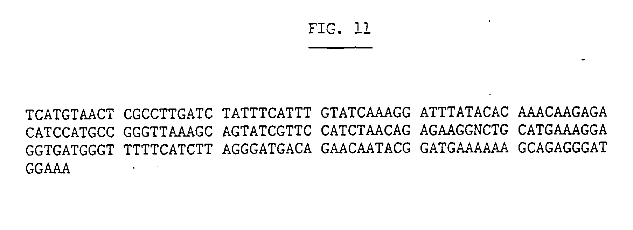

L'invention concerne également le promoteur (SEQ ID NO:26) dérivé du gène qui code pour la xylanase de Bacillus pumilus PRL B12. La séquence du promoteur est illustrée à la figure 11.

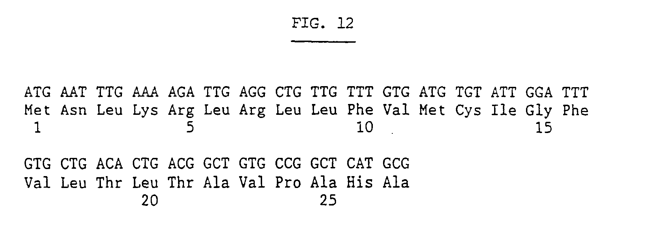

L'invention concerne également la préséquence (SEQ ID NO:27) qui code pour le peptide signal de la xylanase de Bacillus pumilus PRL B12. La séquence corespondante de 27 acides aminés a été identifiée (SEQ ID NO:29). Cette séquence de nucléotides, ainsi que sa traduction en acides aminés, (SEQ ID NO:28) est illustrée à la figure 12.

Le procédé d'obtention et de préparation du promoteur dérivé du gène qui code pour la xylanase de Bacillus pumilus PRL B12 et de la préséquence qui code pour le peptide signal de la xylanase de Bacillus pumilus PRL B12 est décrit à l'exemple 17 et à la figure 1 de la demande de brevet européen 0 634 490.

La souche de Bacillus pumilus PRL B12 a été déposée à la collection ATCC (American Type Culture Collection, 12301 Parklawn Drive, Rockville, Maryland, 20852, Etats-Unis) conformément au Traité de Budapest sous le numéro ATCC 55443 le 24 juin 1993.

L'invention permet l'obtention d'une molécule d'ADN mutée, et la xylanase mutée qui en dérive (pour laquelle la molécule d'ADN mutée code), obtenue par modification de la séquence de nucléotides du gène qui code pour la xylanase définie ci-dessus. Les techniques d'obtention de telles xylanases mutées sont connues de l'homme du métier et sont notamment décrites dans MOLECULAR CLONING - a laboratory manual - SAMBROOK, FRITSCH, MANIATIS - second édition, 1989, chapitre 15.

La présente invention concerne également un vecteur d'expression ou vecteur d'intégration chromosomique contenant une molécule d'ADN, telle que définie ci-dessus. Généralement, le vecteur d'expression ou le vecteur d'intégration chromosomique contient la molécule d'ADN qui comprend la séquence de nucléotides (SEQ ID NO:1) qui code pour la xylanase de Bacillus sp. 720/1. Habituellement, le vecteur d'expression ou le vecteur d'intégration chromosomique contient une molécule d'ADN qui comprend le gène qui code pour la xylanase. De préférence, le vecteur d'expression ou le vecteur d'intégration chromosomique contient la molécule d'ADN qui comprend la séquence de nucléotides (SEQ ID NO:10) qui code pour la xylanase de Bacillus sp. 720/1. De manière particulièrement préférée, ce vecteur est le vecteur d'expression pUBRD-720X11. De bons résultats ont également été obtenus avec le vecteur d'expression pUBR-720X11.

Une variante de l'invention concerne un vecteur d'expression ou un vecteur d'intégration chromosomique qui contient une molécule d'ADN comprenant le gène qui code pour la partie mature de la xylanase. Généralement, le vecteur d'expression ou le vecteur d'intégration chromosomique contient la molécule d'ADN qui comprend la séquence de nucléotides (SEQ ID NO:1) qui code pour la xylanase de Bacillus sp. 720/1. Habituellement, le vecteur d'expression ou le vecteur d'intégration chromosomique contient une molécule d'ADN qui comprend le promoteur dérivé du gène (SEQ ID NO:26) qui code pour la xylanase de Bacillus pumilus PRL B12, une préséquence et la séquence de nucléotides (SEQ ID NO:1) qui code pour la xylanase de Bacillus sp. 720/1. Dans une variante habituelle, le vecteur d'expression contient une molécule d'ADN qui comprend un promoteur, la préséquence (SEQ ID NO:27) qui code pour le peptide signal de la xylanase de Bacillus pumilus PRL B12 et la séquence de nucléotides (SEQ ID NO:1) qui code pour la xylanase de Bacillus sp. 720/1. De préférence, le vecteur d'expression ou le vecteur d'intégration chromosomique contient une molécule d'ADN qui comprend le promoteur dérivé du gène (SEQ ID NO:26) qui code pour la xylanase de Bacillus pumilus PRL B12, la préséquence (SEQ ID NO:27) qui code pour le peptide signal de la xylanase de Bacillus pumilus PRL B12 et la séquence de nucléotides (SEQ ID NO:1) qui code pour la xylanase de Bacillus sp. 720/1. De manière particulièrement préférée, ce vecteur est le vecteur d'expression pBPXD-PRE-720X. De bons résultats ont également été obtenus avec le vecteur d'expression pC-BPX-PRE-720X.

L'invention concerne également un système d'expression utilisable pour la production d'un polypeptide.

Ce système d'expression comprend :
- la séquence du promoteur (SEQ ID NO:26) dérivé du gène qui code pour la xylanase de Bacillus pumilus PRL B12,
- une séquence codant pour un peptide signal, et
- la séquence de nucleotides (SEQ ID NO:1) qui code pour la xylanase de Bacillus sp. 720/1.

Généralement, le système d'expression comprend la séquence d'un terminateur.

Dans une variante, ce système d'expression comprend :
- la séquence d'un promoteur,
- la préséquence (SEQ ID NO:27) qui codé pour le peptide signal de la xylanase de Bacillus pumilus PRL B12, et
- la séquence de nucleotides (SEQ ID NO:1) qui code pour la xylanase de Bacillus sp. 720/1.

Généralement, le système d'expression comprend la séquence d'un terminateur.

Habituellement, ce système d'expression comprend :
- la séquence du promoteur (SEQ ID NO:26) dérivé du gène qui code pour la xylanase de Bacillus pumilus PRL B12,
- la préséquence (SEQ ID NO:27) qui code pour le peptide signal de la xylanase de Bacillus pumilus PRL B12,
- la séquence de nucleotides (SEQ ID NO:1) qui code pour la xylanase de Bacillus sp. 720/1, et
- la séquence d'un terminateur.

La présente invention concerne également des souches recombinantes dans lesquelles le gène codant pour une xylanase est introduit par les techniques de génie génétique. Le gène peut être introduit par le biais d'un vecteur réplicatif ou intégré dans le chromosome de l'hôte en une ou plusieurs copies par le biais d'un vecteur intégratif; la séquence de nucléotides codant pour une xylanase peut être introduite par transformation, soit sous forme intégrée dans l'ADN chromosomique, soit sous forme autoréplicative (plasmide).

L'invention concerne également les souches de microorganismes différentes de l'organisme producteur de départ, dans lesquelles la séquence de nucléotides codant pour une xylanase est introduite par transformation, soit sous forme intégrée dans l'ADN chromosomique, soit sous forme autoréplicative (plasmide), le gène codant pour une xylanase peut être introduit par le biais d'un vecteur réplicatif ou intégré dans le chromosome de l'hôte en une ou plusieurs copies par un vecteur d'intégratif.

L'invention concerne une souche transformée comprenant la molécule d'ADN qui contient le gène de structure qui code pour la xylanase mature de Bacillus sp. 720/1. Généralement, la souche transformée est une souche de bactérie. Habituellement, la souche transformée est choisie parmi les souches d'Escherichia, de Pseudomonas ou de Bacillus. De préférence, la souche transformée est une souche de Bacillus. De manière particulièrement préférée, la souche transformée de Bacillus est une souche de Bacillus licheniformis, une souche de Bacillus pumilus, une souche de Bacillus alcalophilus ou une souche de Bacillus sp. 720/1. De bons résultats ont été obtenus avec une souche de Bacillus licheniformis et avec une souche de Bacillus pumilus.

L'invention concerne la souche transformée de Bacillus comprenant le vecteur d'expression ou le vecteur d'intégration chromosomique qui comprend cette molécule d'ADN. De préférence, la souche transformée de Bacillus est une souche de Bacillus licheniformis. De préférence, également, la souche transformée de Bacillus est une souche de Bacillus sp. 720/1.

La présente invention permet l'obtention d'une xylanase produite par une souche transformée telle que définie ci-dessus.

La présente invention concerne également un procédé pour la production d'une xylanase comprenant la culture d'une bactérie aérobie capable de produire la xylanase dans un milieu nutritif approprié contenant des sources de carbone et d'azote et des sels minéraux sous condition d'aérobiose et la récolte de la xylanase ainsi obtenue. Ce milieu de culture peut être solide ou liquide. De préférence le milieu de culture est liquide. De préférence la bactérie aérobie est une souche de Bacillus ou un dérivé de cette souche capable de produire la xylanase.

La présente invention concerne également un procédé pour la production d'une xylanase comprenant la culture de la souche de Bacillus sp. 720/1 ou un dérivé de cette souche capable de produire la xylanase dans un milieu nutritif approprié contenant des sources de carbone et d'azote et des sels minéraux sous condition d'aérobiose et la récolte de la xylanase ainsi obtenue.

L'invention concerne également un procédé pour la préparation d'une xylanase à partir d'un organisme recombinant, le procédé comprenant l'isolement d'un fragment d'ADN codant pour la xylanase, l'insertion de ce fragment d'ADN dans un vecteur approprié, l'introduction de ce vecteur dans un hôte approprié ou l'introduction de ce fragment d'ADN dans le chromosome d'un hôte approprié, la culture de cet hôte, l'expression de la xylanase et la récolte de la xylanase. L'hôte approprié est choisi généralement parmi le groupe constitué des microorganismes Escherichia coli, Bacillus ou Aspergillus. Habituellement, l'hôte est choisi parmi les Bacillus. De préférence, l'hôte est choisi parmi les microorganismes du genre Bacillus (aérobies). De manière particulièrement préférée, l'hôte est choisi parmi les microorganismes Bacillus subtilis, Bacillus licheniformis, Bacillus alcalophilus, Bacillus pumilus, Bacillus lentus, Bacillus amylolique-faciens ou Bacillus sp. 720/1. De bons résultats ont été obtenus lorsque l'hôte pour l'expression de la xylanase selon la présente invention est une souche recombinante dérivée de Bacillus licheniformis, et de préférence la souche de Bacillus licheniformis SE2 delap1 et la souche de Bacillus licheniformis SE2 delap6. La souche de Bacillus licheniformis SE2 delap1 et la souche de Bacillus licheniformis SE2 delap6 sont décrites dans la demande de brevet européen 0 634 490.

L'invention permet l'obtention d'une xylanase produite, de façon hétérologue, par un microorganisme du genre Bacillus. Habituellement, le microorganisme du genre Bacillus contient un gène codant pour une protéase alcaline lorsqu'il est à l'état sauvage. De préférence ce microorganisme est une souche de Bacillus licheniformis comprenant la molécule d'ADN qui comprend la séquence de nucléotides qui code pour la xylanase de Bacillus sp. 720/1. De manière particulièrement préférée, le gène codant pour la protéase alcaline a été enlevé par délétion de cette souche de Bacillus. Cette souche est de préférence la souche de Bacillus licheniformis SE2 delap1 ou la souche de Bacillus licheniformis SE2 delap6.

Par produite de façon hétérologue, on entend une production qui n'est pas effectuée par le microorganisme naturel, c'est-à-dire le microorganisme qui contient à l'état sauvage le gène qui code pour la xylanase.

Les conditions de culture de ces bactéries telles que composants du milieu nutritif, paramètres de culture, température, pH, aération, agitation, sont bien connues de l'homme du métier. Des exemples de telles techniques sont notamment décrits dans ULLMANN'S ENCYCLOPEDIA OF INDUSTRIAL CHEMISTRY, 1987, 5ième édition, Vol. A9, pages 363-390.

Les techniques de récolte de la xylanase sont bien connues de l'homme du métier et sont choisies selon les utilisations envisagées de la xylanase. Habituellement, on emploie la centrifugation, la filtration, l'ultrafiltration, l'évaporation, la microfiltration, la cristallisation ou une combinaison de l'une ou l'autre de ces techniques telle qu'une centrifugation suivie d'une ultrafiltration. Des exemples de telles techniques sont notamment décrits par R. SCRIBAN, Biotechnologie, (Technique et Documentation Lavoisier), 1982, p. 267-276 et dans ULLMANN'S ENCYCLOPEDIA OF INDUSTRIAL CHEMISTRY, 1987, 5ième édition, Vol. A9, pages 363-390.

La xylanase peut ensuite être purifiée, si nécessaire et selon les utilisations envisagées. Les techniques de purification d'enzymes sont bien connues de l'homme du métier, telles que la précipitation à l'aide d'un sel, tel que le sulfate d'ammonium, ou de solvant, tel que l'acétone ou un alcool. Des exemples de telles techniques sont notamment décrits par R. SCRIBAN, Biotechnologie, (Technique et Documentation Lavoisier), 1982, p. 267-276.

La xylanase peut également être séchée par atomisation ou lyophilisation. Des exemples de telles techniques sont notamment décrits par R. Scriban, Biotechnologie, (Technique et Documentation Lavoisier), 1982, p. 267-276 et dans ULLMANN'S ENCYCLOPEDIA OF INDUSTRIAL CHEMISTRY, 1987, 5ième édition, Vol. A9, pages 363-390.

La présente invention concerne également des compositions enzymatiques comprenant la xylanase selon l'invention et au moins un additif. Ces additifs sont connus de l'homme du métier et sont choisis selon l'utilisation envisagée de la composition. Ils doivent être compatibles avec la xylanase et ne pas affecter, ou peu, l'activité enzymatique de la xylanase. Habituellement, ces additifs sont des stabilisants de l'enzyme, des agents de conservation et des agents de formulation.

Les compositions comprenant la xylanase de la présente invention peuvent être utilisées sous forme solide ou liquide.

La xylanase est formulée selon les utilisations prévues. Des stabilisants ou des agents de conservation peuvent également être ajoutés aux compositions enzymatiques comprenant la xylanase selon l'invention. Par exemple on peut stabiliser la xylanase par l'addition de propylène glycol, d'éthylène glycol, de glycérol, d'amidon, de xylane, d'un sucre tel que du glucose et du sorbitol, d'un sel tel que du chlorure de sodium, du chlorure de calcium, du sorbate de potassium et du benzoate de sodium ou d'un mélange de deux ou plusieurs de ces produits. De bons résultats ont été obtenus avec du propylène glycol. De bons résultats ont été obtenus avec du sorbitol.

La xylanase selon l'invention a des débouchés multiples dans diverses industries, telles que, par exemples, les industries alimentaires, les industries pharmaceutiques ou les industries chimiques.

La xylanase peut notamment être utilisée en boulangerie. Un exemple d'une utilisation d'une xylanase en boulangerie est, en particulier, décrit dans la demande de brevet international WO 94/04664.

La xylanase peut notamment être utilisée pour le traitement de la pâte à papier. Un exemple d'une utilisation d'une xylanase pour le traitement de la pâte à papier est, en particulier, décrit dans la demande de brevet européen 0 634 490. La xylanase de la présente invention est notamment efficace sur la pâte venant du bois d'eucalyptus, comme l'illustre l'exemple 13 de la présente demande de brevet.

La xylanase peut notamment être utilisée en alimentation animale. Un exemple d'une utilisation d'une xylanase en alimentation animale est, en particulier, décrit dans la demande de brevet européen 0 507 723.
La figure 1 (figure 1a et figure 1b) représente la séquence de nucléotides (SEQ ID NO:2) codant pour la xylanase mature ainsi que sa traduction en acides aminés.
La figure 2 (figure 2a et figure 2b) représente la séquence de nucléotides (SEQ ID NO:11) du gène codant pour la xylanase ainsi que sa traduction en acides aminés.
La figure 3 représente la carte de restriction du plasmide pUBR2002.
La figure 4 représente la carte de restriction du plasmide pUBR-720X1.
La figure 5 représente la carte de restriction du plasmide pUBR-720X11.
La figure 6 représente la carte de restriction du plasmide pUBRD-720X 11.
La figure 7 représente la carte de restriction du plasmide pUBC2001.
La figure 8 représente la carte de restriction du plasmide pC-BPX-PRE-2003.
La figure 9 représente la carte de restriction du plasmide pC-BPX-PRE-720X.
La figure 10 représente la carte de restriction du plasmide pBPXD-PRE-720X.
La figure 11 représente le promoteur (SEQ ID NO:26) dérivé du gène qui code pour la xylanase de Bacillus pumilus PRL B12.
La figure 12 représente la préséquence (SEQ ID NO:28) qui code pour le peptide signal de la xylanase de Bacillus pumilus PRL B12.

La signification des abréviations et symboles utilisés dans ces figures est rassemblée dans le tableau suivant.

| Symbole Abréviation | Signification |
|---|---|
| OriEC | origine de réplication dans E. coli |
| REP | protéine nécessaire pour la réplication dans Bacillus |
| Ori+ | origine de réplication du brin + dans Bacillus |
| Ori- | origine de réplication du brin - dans Bacillus |
| AmpR | gène apportant la résistance à l'ampicilline |
| KmR | gène apportant la résistance à la kanamycine |
| BlmR | gène apportant la résistance à la bléomycine |
| 5'720XYL | séquence 5' située en amont de la séquence codant pour la xylanase de Bacillus sp. 720/1 |
| 3'720XYL | séquence 3' située en aval de la séquence codant pour la xylanase de Bacillus sp. 720/1 |
| 720XYL | séquence codant pour le précurseur de la xylanase de Bacillus sp. 720/1 |
| 5'BPUXYL-PRE | promoteur, et site de fixation au ribosome, de la xylanase de Bacillus pumilus PRL B12, suivi de la préséquence de la xylanase de Bacillus pumilus PRL B12. |
| 720XYL-MAT | séquence codant pour la partie mature de la xylanase de Bacillus sp. 720/1 |

La présente invention est illustrée par les exemples suivants.

### Exemple 1

### Isolement et caractérisation de la souche de Bacillus sp. 720/1

La souche de Bacillus sp. 720/1 a été isolée d'un échantillon de sol, prélevé en Argentine, sur un milieu nutritif gélosé et sélectionnée pour sa capacité de dégrader un dérivé coloré du xylane connu sous le nom de l'AZCL-xylane et vendu par la Société MEGAZYME.

Cette souche a été mise en culture à 37 °C dans le milieu de croissance LBS/C dont la composition est la suivante : son de blé 10 g/l, TRYPTONE (DIFCO) 10 g/l, extrait de levure 5 g/l, NaCl 10 g/l, Na₂CO₃ 5,3 g/l, NaHCO₃ 4,2 g/l.

Le carbonate et le bicarbonate de sodium sont stérilisés séparément, puis ajoutés aseptiquement aux autres composants du milieu stérile. Le milieu gélosé contient en outre 20 g/l d'agar. La souche de la présente invention a été identifiée par ses caractéristiques biochimiques : bactérie Gram positif, aérobie qui se présente sous le forme de bâtonnet, elle forme une endo-spore. Elle appartient donc au genre Bacillus.

Les cellules végétatives de cette souche en culture sur le milieu LBS/C gélosé à 37 °C ont une forme de bacille de taille 0,8 x 3,0-5 *µ*m. La mobilité des cellules végétatives est positive.

Après une croissance de 13 jours à 37 °C sur le milieu TSA gélosé, l'observation microscopique révèle la présence de sporanges. Le milieu TSA gélosé contient 15 g/l de TRYPTONE (DIFCO), 5 g/l de peptone de soja; 5 g/l de NaCl et 15 g/l de gélose.

La souche est oligosporogène.

Le test de la catalase est positif en présence de 10 % (v/v) de peroxyde d'hydrogène. Le test de l'oxydase est positif en présence de 1 % (p/v) de tétraméthyl-1,4-phénylène-diamine-dihydrochlorure.

Cette souche est aérobie, c'est-à-dire qu'elle se développe en aérobiose. Elle ne se développe pas en anaérobiose, c'est-à-dire sous une atmosphère de 84 % (v/v) N₂, 8 % (v/v) CO₂, 8 % (v/v) H₂ à 37 °C. L'abréviation % (v/v) représente un pourcentage exprimé en volume par volume.

Cette souche n'est pas thermophile. Elle présente un développement normal après incubation en milieu LBS/C gélosé à 20 °C, 30 °C, 37 °C et 45 ° C, par contre elle ne se développe pas à 50 ° C et 55 ° C, ni à 10 °C.

Elle présente un développement normal après incubation en milieu LBS/C gélosé en présence de NaCl aux concentrations de 2,0 % (p/v) et de 3,5 % (p/v), présente un développement faible en présence de 5,0 % (p/v) et de 7,0 % (p/v) de NaCl. L'abréviation % (p/v) représente un pourcentage exprimé en poids par volume.

La souche de Bacillus sp. 720/1 n'acidifie pas le glucose.

La souche de Bacillus sp. 720/1 a été identifiée au moyen de la galerie API 50 CHB et de la galerie API 20 E en suivant les instructions d'utilisation du fournisseur (API SYSTEM, France). La souche de Bacillus sp. 720/1 utilise le glycérol, le N-acétylglucosamine, l'arbutine, le citrate, la galactose, l'amygdaline, le melibiose et hydrolyse la gélatine. Ces caractéristiques différencient nettement la souche de Bacillus sp. 720/1 d'une souche de Bacillus pumilus. En effet, une souche de Bacillus pumilus ne présente aucune de ces caractéristiques.

La souche de Bacillus sp. 720/1 a également été identifiée au moyen du système BIOLOG (USA). La banque de données analysant les résultats de ce système donne un score de 0,564 pour le Bacillus coagulans, de 0,097 pour le Bacillus subtilis, de 0,057 pour le Bacillus licheniformis et de 0,00 pour le Bacillus pumilus. Ces caractéristiques différencient nettement la souche de Bacillus sp. 720/1 d'une souche de Bacillus coagulans, d'une souche de Bacillus subtilis, d'une souche de Bacillus licheniformis et d'une souche de Bacillus pumilus.

La bactérie isolée appartient donc au genre Bacillus, aucune espèce connue n'a pu être determinée.

La souche de Bacillus sp. 720/1 a été déposée à la collection nommée BELGIAN COORDINATED COLLECTIONS OF MICROORGANISMS (LMG culture collection) sous le numéro LMG P-14798.

### Exemple 2

### Production de la xylanase par le Bacillus sp. 720/1

La souche de Bacillus sp. 720/1 est mise en culture sur boîte de Pétri contenant un milieu LBS/C, gélosé, à 37 °C durant 48 heures (culture A).

Puis, à partir de la culture A, on réalise une culture dans un milieu LB/C liquide dont la composition est identique à celle du milieu LBS/C mais sans son de blé, à 37 °C durant 24 heures sous agitation orbitale à raison de 250 révolutions par minute (culture B) avec une amplitude d'environ 2,54 cm.

500 ml de la culture B sont ensuite transférés dans un fermenteur de 20 1 contenant 14 de milieu LBS/C. Le pH est laissé libre, la vitesse d'agitation et le debit d'air insufflé dans le fermenteur sont tels que la pression partielle en oxygène dissous dans le milieu de culture ne soit pas inférieur à 30 % de la saturation.

Après 72 heures de culture à 37 °C, on sépare la xylanase et la biomasse cellulaire par centrifugation (BECKMAN J21, rotor JA10) à 8 000 tours par minute pendant 30 minutes. La xylanase produite par la souche de Bacillus sp. 720/1 est extracellulaire. Puis on sépare les matières insolubles résiduelles de la xylanase, à partir du surnageant de centrifugation, par microfiltration (cartouche KROS FLOII 0,2 *µ* de porosité, Société MICROGON).

On lave le rétentat de microfiltration avec 1 d'eau déminéralisée. On effectue ce lavage trois fois.

Ensuite, on concentre, environ 20 fois, le perméat de cette microfiltration par ultrafiltration sur une cartouche PALL MICROZA SIP 1013 en polysulfone, ayant un seuil de coupure de 6 kD (Société PALL).

On mesure l'activité enzymatique sur le rétentat (produit R) et sur le perméat (produit P) d'ultrafiltration obtenus.

Une unité enzymatique de la xylanase (IU) est définie comme la quantité d'enzyme qui, à un pH de 8,0, à une température de 50 ° C et en présence de xylane, catalyse la libération d'équivalents glucose à raison de 1 *µ*mole de glucose par minute (*µ*mol/minute).

La mesure de l'activité enzymatique xylanase est réalisée selon le protocole décrit par BAILEY, BIELY et POUTANEN, J. BIOTECHNOLOGY, 1992, 23, pages 257-270; à l'exception que le tampon citrate-phosphate cité par BAILEY et al. a été remplacé par le tampon Tris(hydroxyméthyl)aminométhane-HCl, 50 mM (pH de 8,0).

On ajoute au rétentat d'ultrafiltration (produit R) suffisamment de polyéthylène glycol (polyéthylène glycol de MERCK référence 807490) pour obtenir une concentration de 40 % (p/p). Après solubilisation du polyéthylène glycol, on incube la solution obtenue 30 minutes à 25 °C.

Puis, on centrifuge la solution, contenant le polyéthylène glycol et la xylanase, 10 minutes à 8 000 tours par minute (centrifugeuse BECKMAN J21, rotor JA10). On élimine le surnageant de centrifugation. On ajoute au culot de centrifugation suffisamment d'une solution de NaCl (0,9 % v/v) pour retrouver le volume initial du rétentat mis en oeuvre (produit R).

Ensuite, on ajoute à cette suspension contenant la xylanase et du NaCl suffisamment d'acétone pour arriver à une concentration de 40 % (v/v). On incube cette suspension acétonique durant 45 minutes à 4 °C.

Après cette incubation, on centrifuge cette suspension acétonique 10 minutes à 8 000 tours par minute (BECKMAN J21, rotor JA10).

On conserve le surnageant de centrifugation. A ce surnageant de centrifugation, on ajoute de l'acétone à une concentration de 80 % (v/v). On incube cette suspension acétonique 45 minutes à 4 °C.

Après cette incubation, on centrifuge cette suspension acétonique 10 minutes à 8 000 tours par minute (BECKMAN J21, rotor JA10).

On conserve le culot de centrifugation. Celui-ci est mis en suspension dans un volume suffisant d'une solution de NaCl 0,9 % (v/v) pour être solubilisé (produit N).

### Exemple 3

### Purification de la xylanase

Une fraction du rétentat d'ultrafiltration (produit N) obtenu à l'exemple 2, est conditionnée par passage sur colonne de chromatographie de perméation sur gel (colonne BIO-RAD ECONOPAC 10DG) équilibrée avec un tampon Bis-Tris (bis(2-hydroxyéthyl)imino-tris(hydroxyméthyl)méthane) 20mM, pH 6,2 (tampon A). On obtient ainsi une solution nommée produit X.

1 ml de la solution produit X est alors déposé sur une colonne échangeuse de cation S SEPHAROSE HP 16/10 (PHARMACIA) préalablement équilibré avec le tampon A. Le débit est de 2,5 ml par minute, avec une élution isocratique pendant 10 minutes, suivie d'un gradient de concentration en NaCl (de 10 à 50 minutes, la teneur en NaCl passe de 0 à 0,7 M). Un pic unique est détecté pendant le gradient, correspondant à l'élution de la xylanase.

On recueille les fractions contenant l'activité xylanase (solution A).

On vérifie que ces fractions contiennent de la xylanase en déposant 10 µl de chaque fraction sur un milieu gélosé comprenant du xylane (milieu contenant 0,5 g/l d'AZCL-xylane, tampon TRIS 50 mM (pH = 8,0) et 15 g/l d'agar). Un halo se forme autour des fractions qui contiennent de la xylanase.

### Exemple 4

### Séquence d'acides aminés

La séquence d'acides aminés de la xylanase de la présente invention est indirectement déterminée à partir de la séquence de nucléotides (SEQ ID NO:10) du gène qui code pour cette xylanase, dont l'obtention est décrite à l'exemple 14. Ceci est réalisé à l'aide du programme d'ordinateur IntelliGenetics Suite Software for Molecular Biology (Release #5.4) d'IntelliGenetics, Inc. USA. La figure 2 (figure 2a et figure 2b) représente la séquence de nucléotides (SEQ ID NO:10) du gène codant pour la xylanase ainsi que sa traduction en acides aminés (SEQ ID NO:11).

La xylanase est synthétisée sous forme d'un précurseur. Le précurseur de la xylanase contient 248 acides aminés (SEQ ID NO:6). On identifie la séquence de nucléotides (SEQ ID NO:4) codant pour le précurseur de la xylanase ainsi que sa traduction en acides aminés (SEQ ID NO:5).

On identifie la préséquence de la xylanase synthétisée sous forme d'un précurseur. Il s'agit d'une séquence de 27 acides aminés (SEQ ID NO:9). On identifie la séquence de nucléotides correspondante (SEQ ID NO:7).

Ensuite, on identifie la séquence en acides aminés de la xylanase mature. La xylanase mature contient 221 acides aminés (SEQ ID NO:3).

La figure 1 (figure 1a et figure 1b) représente la séquence de nucléotides (SEQ ID NO:1) codant pour la xylanase mature ainsi que sa traduction en acides aminés (SEQ ID NO:2).

### Exemple 5

### Distribution en acides aminés

La distribution en acides aminés de la xylanase mature, déterminée à partir de la séquence en acides aminés (SEQ ID NO:3) de la xylanase (exemple 4), est résumée dans le tableau 1.

**TABLEAU 1**

| Symbole | Acides aminés | Nombre | % (en poids moléculaire) |
|---|---|---|---|
| N | asparagine | 25 | 11,6 |
| Y | tyrosine | 13 | 8,6 |
| T | thréonine | 18 | 7,4 |
| S | serine | 19 | 6,7 |
| I | isoleucine | 14 | 6,4 |
| V | valine | 14 | 5,6 |
| G | glycine | 24 | 5,5 |
| W | tryptophane | 7 | 5,3 |
| K | lysine | 10 | 5,2 |
| R | arginine | 8 | 5,1 |
| Q | glutamine | 9 | 4,7 |
| D | acide aspartique | 10 | 4,7 |
| L | leucine | 10 | 4,6 |
| E | acide glutamique | 8 | 4,2 |
| F | phénylalanine | 7 | 4,2 |
| P | proline | 7 | 2,8 |
| M | méthionine | 5 | 2,7 |
| A | alanine | 8 | 2,3 |
| H | histidine | 4 | 2,2 |
| C | cystéine | 1 | 0,4 |
| B | acide aspartique/asparagine | 0 | 0,0 |
| X | inconnu | 0 | 0,0 |
| Z | glutamine acide glutamique | 0 | 0,0 |

### Exemple 6

### Estimation du poids moléculaire

On estime, par calcul, le poids moléculaire de la xylanase à partir de la séquence en acides aminés de la forme mature de la xylanase, comme décrit à l'exemple 4.

On déduit par calcul un poids moléculaire de 24698,61 Daltons.

### Exemple 7

### Détermination du poids moléculaire

On effectue sur la solution A contenant la xylanase, telle qu'obtenue à l'exemple 3, une concentration sur un dispositif CENTRICON 10 kD (AMICON).

100 µl de la solution concentrée sont déposés sur une colonne de chromatographie de perméation sur gel SUPERDEX 75 HR 10/30 (PHARMACIA). La colonne a été préalablement calibrée au moyen des marqueurs de poids moléculaire (PHARMACIA) GEL FILTRATION LMW calibration kit code 17-0442-01. L'élution a lieu à 0,25 ml/minute au moyen d'un tampon CAPSO (acide 3-(cyclohexylamino)-2-hydroxy-1-propane sulfonique) 25mM, pH 9,2 additionné de NaCl 0,2M.

Le chromatogramme obtenu montre un pic unique correspondant à l'activité xylanase. On en déduit un poids moléculaire apparent de la protéine d'environ 13,5 kD.

On effectue également sur la fraction issue de ce pic unique une électrophorèse en gel de polyacrylamide en conditions dénaturantes (SDS-PAGE). Le système de gel utilisé est le système PHASTSYSTEM de PHARMACIA LKB BIOTECHNOLOGY, avec des gels contenant un gradient de polyacrylamide de 10-15 % (v/v). Les conditions d'électrophorèse sont celles prescrites par le fournisseur. On utilise comme témoin les marqueurs de poids moléculaire PHARMACIA LMW (Low Molecular Weight), référence 17-0446-01. Les marqueurs mis en oeuvre sont la phosphorylase b (94 kD), l'albumine (67 kD), l'ovalbumine (43 kD), la carboanhydrase (30 kD), l'inhibiteur de la trypsine (20,1 kD) et l'alpha-lactalbumine (14,4 kD).

Une coloration au bleu de Coomassie fait apparaître un polypeptide d'un poids moléculaire d'environ 25,7 kD.

### Exemple 8

### Estimation du point isoélectrique

On estime le point isoélectrique de la xylanase à partir de la séquence en acides aminés de la forme mature de la xylanase, comme décrit à l'exemple 4.

Le point isoélectrique estimé est représentatif de la charge nette de la protéine sous forme dénaturée.

On déduit un point isoélectrique de 7,46 pour la xylanase sous forme dénaturée.

### Exemple 9

### Détermination du point isoélectrique

On dépose une fraction de la solution A, telle qu'obtenue à l'exemple 3, sur une colonne de chromatofocalisation MONO P 5/20 (PHARMACIA) en suivant les recommandations du fournisseur, et préalablement équilibrée avec un tampon diéthanolamine 25 mM, pH 9,9.

La colonne est éluée au moyen de la solution d'ampholytes POLYBUFFER 96 (PHARMACIA) diluée 10 fois dans l'eau déminéralisée.

Le pH de la fraction contenant l'activité xylanase est de 9,5.

On vérifie que cette fraction contient la xylanase en déposant 10 µl de celle-ci sur un milieu gélosé comprenant du xylane (milieu contenant 0,5 g/l d'AZCL-xylane, tampon TRIS 50 mM (pH = 8,0) et 15 g/l d'agar). Un halo, qui se présente sous la forme d'une zone d'hydrolyse de l'AZCL-xylane, se forme autour de la fraction qui contient la xylanase.

On réalise sur une fraction de la solution A, telle qu'obtenue à l'exemple 3, une focalisation isoélectrique.

Pour réaliser cela, on réhydrate le gel PHARMACIA DRYIEF par un mélange constitué de 2 ml d'eau déminéralisée, de 150 *µ*l de produit BIOLYTE 8-10 (BIORAD) et de 75 *µ*l de produit PHARMALYTE 8-10,5 (PHARMACIA). On dépose environ 200 nanogrammes de protéines (fraction de la solution A) sur le gel. On suit le protocole décrit par le fournisseur.

On en déduit que la xylanase a un point isoélectrique légèrement supérieur à 9,6, point isoélectrique du marqueur ayant le point isoélectrique le plus élevé mis en oeuvre.

Le point isoélectrique observé expérimentalement est représentatif de la charge de surface de la protéine sous sa forme native.

### Exemple 10

### Profil d'activité en fonction du pH pour la xylanase produite par la souche naturelle (Bacillus sp. 720/1)

On mesure, selon la méthode décrite à l'exemple 2, l'activité enzymatique de la xylanase en présence de xylane (ROTH, référence 7500, xylane de bouleau), à une température de 50 ° C et à différents pH (de 5,6 à 10,35) dans différents tampons choisis pour obtenir le pH souhaité. On met en oeuvre la solution comprenant la xylanase, telle qu'obtenue à l'exemple 2 (produit N).

Les résultats sont rassemblés dans le tableau 2. On note que la marge d'erreur est estimée à environ 25 % dans ce type de mesure.

Au cours de cet essai, l'activité enzymatique maximale a été mesurée pour l'échantillon placé à un pH d'environ 6,2 et à une température d'environ 50 °C durant 15 minutes. Par définition on a donc attribué à cet échantillon une activité enzymatique relative de 100 %.

Cet exemple montre que la xylanase selon l'invention développe une activité enzymatique importante dans une gamme de pH compris entre environ 5,6 et environ 10.

**TABLEAU 2**

| pH | Tampon utilisé (50 mM) | Activité relative % |
|---|---|---|
| 5,6 | Tris-maléate | 85 |
| 6,2 | Tris-maléate | 100 |
| 6,8 | Tris-maléate | 96 |
| 7,5 | Tris-maléate/Tris * | 88 |
| 8,7 | Tris/Capso * | 92 |
| 9,5 | Capso/Caps * | 76 |
| 10 | Caps | 50 |
| 10,35 | Caps | 19 |
| Tris = tris(hydroxyméthyl)aminométhane | | |
| Tris-maléate = tampon formé de tris(hydroxyméthyl)aminométhane (50 mM) et d'acide maléique (50 mM) dont la proportion entre les composants est choisie en fonction du pH souhaité, le pH étant ajusté au moyen de NaOH (1 M). | | |
| Capso = acide 3-(cyclohexylamino)-2-hydroxy-1-propane sulfonique | | |
| Caps = acide 3-(cyclohexylamino)-1-propane sulfonique | | |

Le symbole * signifie que la valeur obtenue est la moyenne des mesures effectuées au même pH mais obtenues avec les deux tampons.

Cet exemple montre que la xylanase selon l'invention développe une activité enzymatique élevée dans une très large gamme de pH.

### Exemple 11

### Effet du pH sur l'activité de la xylanase produite par la souche de Bacillus sp. 720/1 en aide au blanchiment de la pâte de résineux

On prépare trois suspensions aqueuses d'une pâte de pin (en provenance de la société SCA) ayant 2,5 % (en poids de matière sèche) de consistance et ayant un indice Kappa initial de 17.

On ajuste le pH de ces suspensions à pH 5 avec H₂SO₄.

### 1e étape : étape à l'enzyme (étape X)

On dilue la solution nommée produit N, telle qu'obtenue à l'exemple 2, avec de l'eau déminéralisée pour obtenir une solution d'enzyme ayant une activité enzymatique de 25 UI/ml (comme décrit à l'exemple 2).

On ajoute cette solution d'enzyme, contenant la xylanase, à une suspension de pâte de pin de telle sorte que la suspension de pâte soit traitée au moyen de 5 UI/g de pâte sèche

Aux deux autres suspensions de pâte de pin, on ajoute de l'eau déminéralisée à la place de la solution d'enzyme dans des proportions identiques.

Puis on incube les trois suspensions pendant 2 heures à 50°C sans agitation.

### 2e étape : étape au chlore (étape C)

Ensuite, chaque suspension de pâte, ainsi obtenue, est soumise à un traitement de blanchiment qui consiste en une chloration avec de l'eau chlorée. Ce traitement a lieu sur une pâte ayant une consistance de 3 % en poids de matière sèche.

Pour cela, on ajoute à la suspension de pâte traitée enzymatiquement et à une suspension de pâte non traitée enzymatiquement une quantité de chlore de 2,89 (= 0,17 x 17) % (poids/poids de pâte sèche). On ajoute à l'autre suspension de pâte non traitée enzymatiquement une quantité de chlore de 3,40 (0,20 x 17) % (poids/poids).

On incube les 3 suspensions pendant 1 heure à température ambiante. Puis, on lave la pâte avec 40 volumes d'eau déminéralisée.

### 3e étape : étape à la soude (étape E)

Ensuite, on effectue une extraction alcaline qui consiste à ajouter 2 % (poids/poids de pâte sèche) de NaOH aux trois suspensions obtenues ci-dessus et qui ont une consistance de 5 % en poids de matière sèche.

On incube les trois suspensions pendant 1 heure 30 à une température de 60 °C. Puis, on lave la pâte avec 40 volumes d'eau déminéralisée et on la récupère sous forme de feuille à une blancheur d'environ 45 ° ISO (+/- 3° ISO).

On mesure l'indice Kappa des trois feuilles obtenues.

L'indice Kappa est relatif à la mesure de la quantité de lignine présente dans la pâte. L'indice Kappa est un nombre qui représente le volume (en millilitre) d'une solution de permanganate de potassium 0,1 N (KMnO₄) consommé par un gramme de pâte sèche dans les conditions spécifiées et en suivant les procédures décrites dans la norme TAPPI (Technical Committee of the Association of the Pulp and Paper industry) #T236cm-85 (1985).

Le degré ISO est relatif à la mesure de brillance du papier obtenu à partir de la pâte. Cette valeur est un facteur de la réflectance du papier obtenu à partir de la pâte dans les conditions spécifiées et en suivant les procédures décrites dans la norme ISO (The International Organization for Standardization) #2469 publiée dans la norme #ISO 2470-1977 (F) en complément de la norme #2470.

On réalise quatre essais identiques à l'exception de la mise à pH initiale à pH 5. En effet, on réalise quatre essais avec une suspension aqueuse de pâte de pin dont le pH a été ajusté respectivement à pH 6, à pH 7, à pH 8, à pH 9 au lieu de pH 5.

Les résultats sont rassemblés dans le tableau 3.

**TABLEAU 3**

| | | | |
|---|---|---|---|
| Quantité d'enzymes mises en oeuvre (UI/g) | 5 | 0 | 0 |
| Quantité de chlore mis en oeuvre (% en poids/poids de pâte sèche) | 2,89 | 2,89 | 3,40 |
| pH initial | Indice Kappa | | |
| 5 | 4,63 | 5,01 | 4,18 |
| 6 | 3,81 | / | 4,11 |
| 7 | 3,81 | 5,01 | 4,06 |
| 8 | 3,55 | / | 4,21 |
| 9 | 3,71 | 4,91 | 4,07 |

Le symbole / signifie que la suspension de pâte n'a pas été testée.

Ces résultats montrent que la xylanase selon l'invention permet une réduction d'environ 15 à 20 % de la quantité de chlore pour une pâte blanchie à 45 ° ISO. De plus, ces résultats sont obtenus aussi bien à un pH alcalin qu'à un pH acide. Ces bons résultats sont également obtenus à un pH d'environ 9.

Cet exemple montre que la xylanase selon l'invention présente une activité dans une large gamme de pH. En effet, la xylanase selon l'invention est active dans une gamme de pH compris entre environ 5 et environ 10. Elle est particulièrement active pour des valeurs de pH supérieures ou égales à environ 6. Elle est particulièrement active pour des valeurs de pH inférieures ou égales à environ 9.

### Exemple 12

### Effet de la température sur l'activité de la xylanase produite par la souche de Bacillus sp. 720/1 en aide au blanchiment de la pâte de résineux

On reproduit l'exemple 11 avec 5 suspensions de pâte de résineux à pH 8. L'étape de traitement enzymatique est réalisée à un pH de 8 et à différentes températures (55, 60 et 65°C).

Les résultats sont rassemblés dans le tableau 4.

**TABLEAU 4**

| | | | |
|---|---|---|---|
| Quantité d'enzymes mises en oeuvre (UI/g) | 5 | 0 | 0 |
| Quantité de chlore mis en oeuvre (% en poids/poids de pâte sèche) | 2,89 | 2,89 | 3,40 |
| Température °C | Indice Kappa | | |
| 55 | 3,89 | / | / |
| 60 | 3,64 | 4,77 | 4,09 |
| 65 | 3,49 | / | / |

Le symbole / signifie que la suspension de pâte n'a pas été testée.

On observe que la pâte est blanchie à environ 45° ISO.

Ces résultats montrent que l'indice Kappa des échantillons de pâte traitée enzymatiquement reste largement inférieur à l'indice de l'échantillon non traité enzymatiquement.

Cet exemple montre que la xylanase selon l'invention est active dans une large gamme de température. Elle est active à une température d'environ 65°C.

Cet exemple montre également que la xylanase selon l'invention est stable à une température d'environ 60°C.

### Exemple 13

### Activité de la xylanase produite par la souche de Bacillus sp. 720/1 en aide au blanchiment de la pâte d'eucalyptus dans la séquence ECF

Pour cet exemple, on met en oeuvre une pâte d'eucalyptus venant de la société CEASA MILL (Espagne). La pâte est traitée en suivant une séquence ECF ("Elemental Chlorine Free"), c'est-à-dire que la succession d'étapes constituant la séquence ne fait pas appel à du chlore élémentaire.

### 1e étape : étape à l'oxygène (étape O)

La pâte est traitée par un procédé mettant en oeuvre de l'oxygène tel que décrit dans le brevet U.S. 4,462,864, de telle sorte qu'une pâte ayant un indice Kappa initial de 12,3 et un degré ISO initial de 33,4 est obtenue.

Deux suspensions aqueuses sont préparées à partir de cette pâte traitée par l'oxygène ayant une consistance de 4 % en poids de matière sèche.

On ajuste le pH de ces suspensions à pH 9 avec HCl.

### 2e étape : étape à l'enzyme (étape X)

On dilue la solution nommée produit N, telle qu'obtenue à l'exemple 2, avec de l'eau déminéralisée pour obtenir une solution d'enzyme ayant une activité enzymatique de 25 UI/ml.

On ajoute cette solution d'enzyme, contenant la xylanase, à une suspension de pâte d'eucalyptus de telle sorte que la suspension de pâte soit traitée au moyen de 10 UI/g de pâte sèche.

Aux deux autres suspensions de pâte d'eucalyptus, on ajoute de l'eau déminéralisée à la place de la solution d'enzyme.

Puis on incube les 3 suspensions pendant 1 heure 30 minutes à 50°C sans agitation.

### 3e étape : étape au dioxyde de chlore (étape D)

Ensuite, chaque suspension de pâte, ainsi obtenue, est soumise à un traitement de blanchiment qui consiste en une chloration avec du dioxyde de chlore. Ce traitement a lieu sur une pâte ayant une consistance de 3 % en poids de matière sèche.

Pour cela, on ajoute à la suspension de pâte traitée enzymatiquement et à une suspension de pâte non traitée enzymatiquement une quantité de dioxyde de chlore de 0,6 % (poids/poids de pâte sèche). On ajoute à l'autre suspension de pâte non traitée enzymatiquement une quantité de dioxyde de chlore de 1 % (poids/poids).

On incube les 3 suspensions pendant 30 minutes à 50 °C. Puis, on lave la pâte avec 40 volumes d'eau déminéralisée.

### 4e étape : étape à la soude et au peroxyde d'hydrogène (étape E/P).

Ensuite, on effectue une extraction alcaline qui consiste à ajouter 1,8 % (poids/poids de pâte sèche) de NaOH et 0,5 % (poids/poids de pâte sèche) de peroxyde d'hydrogène aux trois suspensions obtenues ci-dessus et qui ont une consistance de 12 % en poids de matière sèche.

On incube les trois suspensions pendant 1 heure 30 à une température de 70 °C. Puis, on lave la pâte avec 40 volumes d'eau déminéralisée.

### 5e étape : étape au dioxyde de chlore (étape D)

Ensuite, chaque suspension de pâte, ainsi obtenue, est soumise de nouveau à un traitement de blanchiment qui consiste en une chloration avec du dioxyde de chlore. Ce traitement a lieu sur une pâte ayant une consistance de 12%.

On ajoute à ces trois suspensions une quantité de dioxyde de chlore de 0,5 % (poids/poids de pâte sèche).

On incube les 3 suspensions pendant 2 heures à 75 °C. Puis, on lave la pâte avec 40 volumes d'eau déminéralisée.

### 6e étape : étape à la soude et au peroxyde d'hydrogène (étape E/P)

Ensuite, on effectue une extraction alcaline qui consiste à ajouter 0,6 % (poids/poids de pâte sèche) de NaOH et 0,3 % (poids/poids de pâte sèche) de peroxyde d'hydrogène aux trois suspensions obtenues ci-dessus et qui ont une consistance de 12 % en poids de matière sèche.

On incube les trois suspensions pendant 1 heure 30 à une température de 70 °C. Puis, on lave la pâte avec 40 volumes d'eau déminéralisée.

### 7e étape : étape au dioxyde de chlore (étape D)

Ensuite, chaque suspension de pâte, ainsi obtenue, est soumise à un traitement de blanchiment qui consiste en une chloration avec du dioxyde de chlore. Ce traitement a lieu sur une pâte ayant une consistance de 12 %.

On ajoute à ces trois suspensions une quantité de dioxyde de chlore de 0,3 % (poids/poids).

On incube les 3 suspensions pendant 2 heures 30 minutes à 75°C. Puis, on lave la pâte avec 40 volumes d'eau déminéralisée et on la récupère sous forme de feuille.

On mesure le degré ISO des trois feuilles obtenues.

Les résultats sont rassemblés dans le tableau 5.

**TABLEAU 5**

| | | | |
|---|---|---|---|
| Quantité d'enzymes mises en oeuvre à l'étape 2 en UI/g | 10 | 0 | 0 |
| Quantité de dioxyde de chlore mis en oeuvre à l'étape 3 en % (poids/poids de pâte sèche) | 0,6 | 0,6 | 1,0 |
| °ISO | 88,5 | 85,5 | 87,9 |

Cet exemple montre que la xylanase selon l'invention est efficace sur la pâte d'eucalyptus. De plus, elle ne nécessite aucun ajustement de pH, puisqu'elle présente l'avantage d'être active au pH de la pâte, c'est-à-dire à un pH d'environ 9. Cet exemple montre que la xylanase selon l'invention est une xylanase alcaline.

Cet exemple montre également qu'en comparaison de ce qui est obtenu sans xylanase, l'utilisation de la xylanase selon l'invention entraîne une augmentation de la brillance d'au moins 3 ° ISO pour une quantité de ClO₂ fixée.

Cet exemple montre également que l'utilisation de la xylanase selon l'invention permet de réduire la quantité de ClO₂ d'environ 4 à 5 kg/tonne de pâte, ce qui représente environ 25 à 30 % de la quantité totale de ClO₂ nécessaire.

### Exemple 14

### Détermination de la séquence nucléotidique et protéique de la xylanase de Bacillus sp. 720/1

### 1. Extraction de l'ADN chromosomique à partir de la souche de Bacillus sp. 720/1

A partir de la culture B, telle qu'obtenue à l'exemple 2, une culture de 200 ml de la souche de Bacillus sp. 720/1 est réalisée en milieu LB/C durant 16 heures à 37 °C. Le milieu LB/C est identique au milieu LBS/C, décrit à l'exemple 1, sans l'addition de son de blé.

Lorsque cette culture est réalisée et qu'elle est en phase stationnaire, elle est centrifugée (BECKMAN J 21, rotor JA10) à 5 000 tours par minute pendant 10 minutes. Le culot de centrifugation ainsi obtenu est repris dans 9 ml de tampon TRIS-HCl (tris(hydroxyméthyl)aminométhane acidifié avec HCl 0,1 M), à un pH de 8, EDTA (acide éthylènediaminetétraacétique) 0,1 M, NaCl 0,15 M contenant 18 mg de lysozyme, la suspension ainsi obtenue est incubée 15 minutes à 37°C.

Le lysat ainsi obtenu est ensuite traité par 200 µl d'une solution de RNAse à 10 mg/ml pendant 20 minutes à 50 °C. 1 ml d'une solution de SDS (sodium dodécyl sulfate) à 10 % (p/v) est alors ajoutée à ce lysat. Ensuite on incube ce lysat durant 30 minutes à 70°C.

Puis le lysat est refroidi aux environs de 45 °C, on y ajoute ensuite 0,5 ml d'une solution de protéinase K (vendue par BOEHRINGER Mannheim) à 20 mg/ml (préparée extemporanément).

Le lysat est incubé à 45 ° C sous agitation jusqu'à l'obtention d'une solution transparente.

On effectue à partir de cette solution transparente plusieurs extractions au phénol sous les conditions et en suivant les procédures décrites dans Molecular Cloning - a laboratory manual - SAMBROOK, FRITSCH, MANIATIS - second edition, 1989, à la page E.3, jusqu'à l'obtention d'une interface propre, comme cela y est décrit.

L'ADN est précipité par 20 ml d'éthanol. Le précipité est récupéré par centrifugation à 5 000 tours par minute (BECKMAN J21, rotor JA10) pendant 5 minutes, puis mis en suspension dans 2 ml de tampon TE à pH 8,0 (10 mM TRIS-HCl, 1mM EDTA à pH 8,0). Cette suspension contient l'ADN chromosomique.

### 2. Construction du vecteur pUBR2002

Le vecteur pUBR2002 (E. coli - Bacillus subtilis) a été obtenu à partir du plasmide pBR322 qui est vendu par la société BIOLABS (CLONTECH LABORATORIES catalogue n° 6210-1) et du vecteur pUB131.

On construit deux oligonucléotides synthétiques par la technique décrite dans BEAUCAGE et al. (1981), Tetrahedron Letters, 22, pages 1859-1882 et en utilisant des β-cyanoéthyl phosphoramidites dans un appareil de BIOSEARCH CYCLONE SYNTHESIZER.

Les séquences de ces deux oligonucléotides sont les suivantes:

Ces deux oligonucléotides sont utilisés pour effectuer une amplification par PCR à partir du plasmide pBR322 selon la technique décrite dans Molecular Cloning, a laboratory Manual - SAMBROOK et al., second edition, 1989, pages 14.18-14.19.

Le fragment amplifié par PCR contient le réplicon E. coli limité de part et d'autre par les sites de restriction AvrII, BstEII, NotI, SfiI, SnabI.

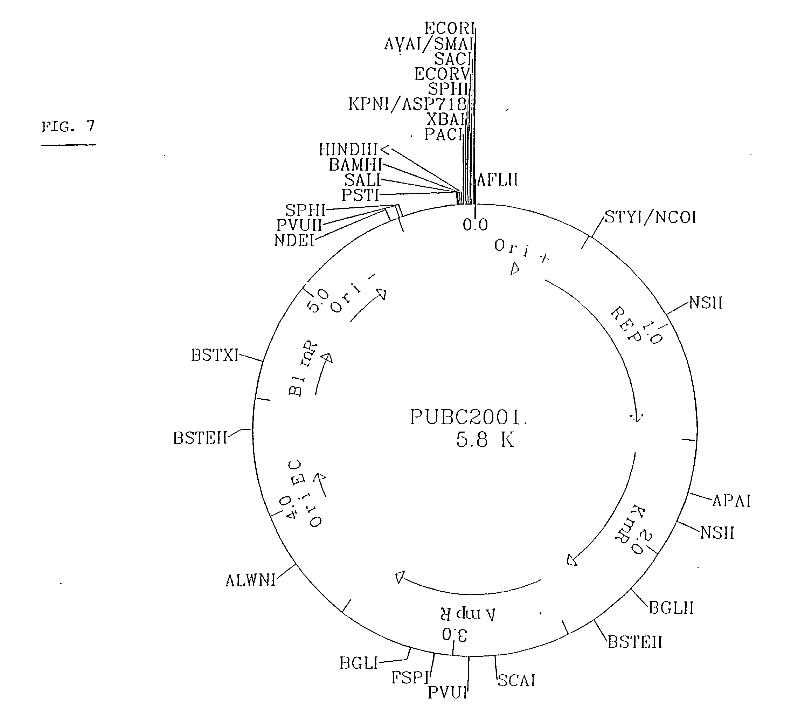

Le fragment SnabI-SnabI d'environ 2,8 kpb (kpb = 1000 paires de bases) est ligaturé avec le vecteur pUB131 qui a préalablement fait l'objet d'une digestion avec SnabI. La construction du vecteur pUB131 est décrite . à l'exemple 10 et à la figure 7 du brevet Etats-Unis 5,352,603 (demande de brevet européen 0 415 296).

La technique de la ligation est décrite par SAMBROOK et al (pages 1.68-1.69). Toutes les ligations réalisées dans les exemples de cette demande ont été effectuées en suivant cette technique.

La ligation ainsi obtenue est transformée dans des cellules d'E. coli MC1061 [CLONTECH LABORATORIES, catalogue n° C-1070-1] par électroporation (SAMBROOK et al, pages 1.75-1.81). Les cellules transformées sont mises en culture sur boîte de Pétri contenant le milieu LB gélosé, 100 *µ*g/ml d'ampicilline et 10 *µ*g/ml de kanamycine, à 37°C pendant environ 18 heures.

Les plasmides sont extraits des colonies isolées par la méthode de la lyse alcaline (SAMBROOK et al., pages 1.25-1.28) et sont soumis à une analyse par restriction, analyse décrite dans Molecular Cloning, a laboratory Manual - MANIATIS et al., 1982, Cold Spring Harbor Laboratory, pages 374-379.

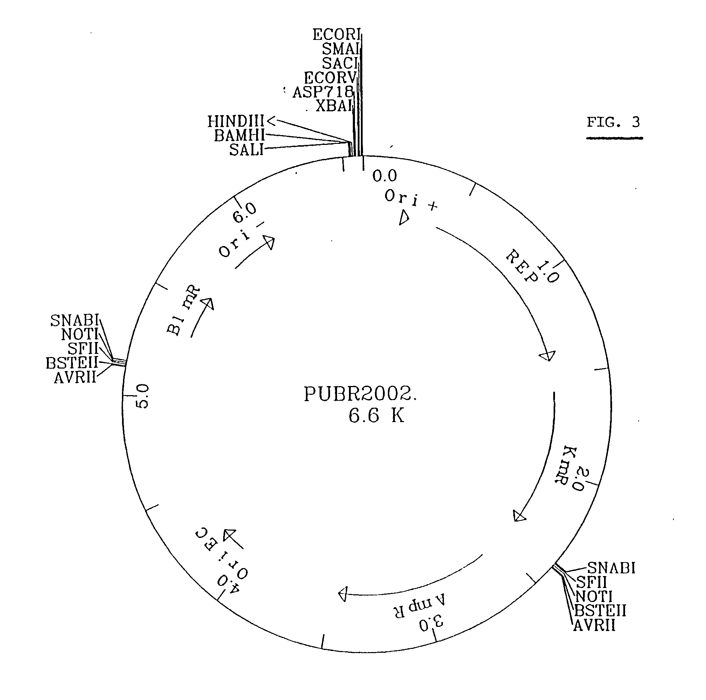

On obtient une souche dont on extrait le vecteur qui est dénommé pUBR2002 (figure 3).

### 3. Construction d'une collection de gènes de Bacillus sp. 720/1

A partir de la suspension le contenant, l'ADN chromosomique est clivé partiellement par l'enzyme de restriction Sau3AI. Les conditions de restriction sont celles décrites par SAMBROOK et al. (pages 5.28-5.32), à part que ces conditions de restriction sont augmentées d'un facteur 10, afin d'obtenir une quantité d'ADN suffisante pour les étapes de purification suivantes.

Le rapport entre la quantité d'ADN mise en oeuvre et la quantité d'enzyme est ajusté afin d'obtenir un maximum de fragments de taille comprise entre 4 et 7 kpb (kpb : 10³ paires de bases).

L'ensemble des fragments ainsi obtenus est ensuite soumis à une électrophorèse en gel d'agarose (0,8 % p/v), comme décrit par SAMBROOK et al., pages 6.01-6.19, et les fragments de taille comprise entre 4 et 7 kpb sont isolés et purifiés par la méthode de filtration suivie d'une centrifugation décrite dans ZHU et al., Bio/Technology 3, 1985, pages 1014-1016.

Les fragments d'ADN ainsi purifiés sont ensuite ligaturés (selon la méthode décrite par SAMBROOK et al. (pages 1.68-1.69) avec le plasmide pUBR2002 (E. coli - Bacillus subtilis), préalablement coupé au site BamHI et déphosphorylé comme décrit par SAMBROOK et al. (pages 1.60-1.61).

La ligation ainsi obtenue est utilisée pour transformer des cellules de E. coli MC1061 par électroporation (SAMBROOK et al., pages 1.75-1.81).

### 4. Criblage de la collection de gènes

Les cellules transformées de E. coli sont mises en culture sur boîte de Pétri contenant le milieu LB gélosé, 0,8 g/l d'AZCL-xylane et 100 µg/ml d'ampicilline, durant environ 24 heures à 37 °C. On isole une colonie présentant une zone d'hydrolyse.

Le plasmide présent dans cette colonie est extrait et isolé par la technique de la lyse alcaline décrite dans SAMBROOK et al., pages 1.25-1.28.

On effectue une analyse par restriction (SAMBROOK et al., page 1.85). Cette analyse indique que le fragment d'ADN obtenu qui contient le gène de la xylanase a une taille d'environ 3,5 kpb (kpb = 1 000 paires de bases). Il est porté par le vecteur pUBR2002 qui a été ligaturé.

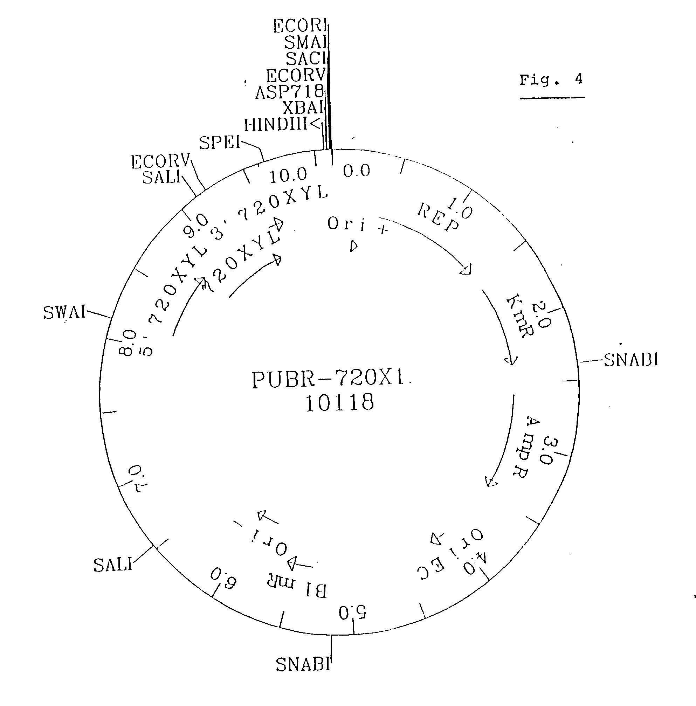

On obtient ainsi le plasmide pUBR-720X1 (figure 4).

### 5. Clonage d'un fragment chromosomique contenant le gène de la xylanase

Le plasmide pUBR-720X1 est digéré par des enzymes de restriction aux sites SwaI et SpeI. Le gène de la xylanase est ainsi obtenu sur un fragment d'ADN SwaI-SpeI d'environ 1,5 kpb.

Ce fragment d'ADN SwaI-SpeI est soumis à un traitement avec le fragment Klenow de l'ADN polymérase (SAMBROOK et al., pages F.2-F.3).

La préparation d'ADN ainsi obtenue est ligaturée avec le vecteur pUBR2002, qui a été préalablement digéré avec EcoRV et déphosphorylé.

La ligation est ensuite transformée dans des cellules d'E. coli MC1061 par électroporation.

Les souches transformées sont sélectionnées sur boîte de Pétri contenant le milieu LB (Luria-Bertani) gélosé, 0,8 g/l d'AZCL-xylane (MEGAZYME) et 100 *µ*g/ml d'ampicilline, après croissance à 37 ° C pendant environ 24 heures.

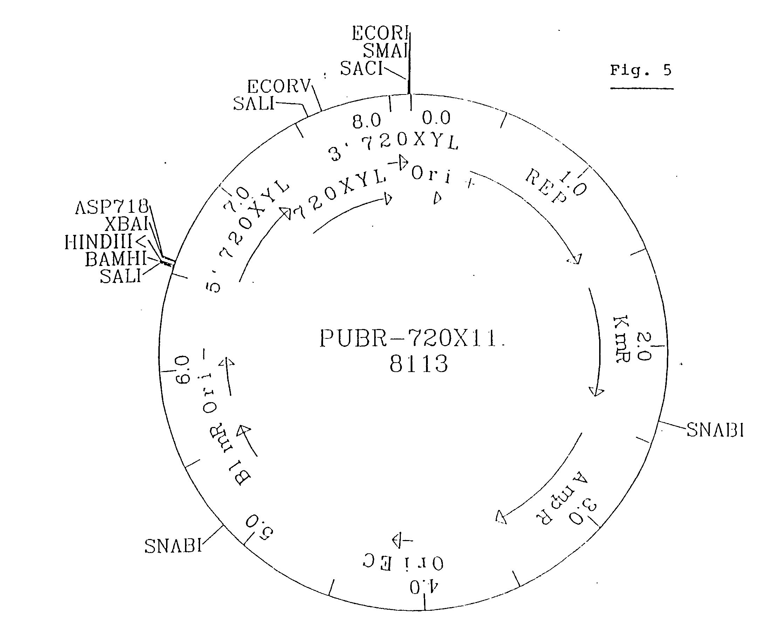

Les colonies présentant une zone d'hydrolyse sont isolées. Les plasmides sont extraits des colonies isolées par la technique de la lyse alcaline (SAMBROOK et al., pages 1.25-1.28) et sont soumis à une analyse par restriction (MANIATIS et al., 1982, pages 374-379). Cette analyse par restriction montre que le plasmide isolé, pUBR-720X11 (figure 5), contient le gène de la xylanase sur un fragment d'environ 1,5 kpb de l'ADN chromosomique du Bacillus sp. 720/1.

Le plasmide pUBR-720X11 est ensuite utilisé pour transformer les cellules d'E. coli JM109 (CLONTECH LABORATORIES catalogue n° C1005-1) par la technique au CaCl₂ (SAMBROOK et al., pages 1.82-1.84).

Les cellules transformées de E. coli sont cultivées sur boîte de Pétri contenant le milieu LB gélosé, 0,8 g/l d'AZCL-xylane et 100 *µ*g/ml d'ampicilline. Après croissance à 37 °C pendant environ 24 heures, on observe une zone d'hydrolyse autour des colonies. Ceci montre que les cellules transformées de E. coli expriment bien la xylanase.

La technique permettant de déphosphoryler les fragments d'ADN ou de linéariser les vecteurs est décrite par SAMBROOK et al. (pages 1.60-1.61).

On isole une colonie présentant une zone d'hydrolyse. Le plasmide présent dans cette colonie est extrait et isolé par la technique de la lyse alcaline.

On établit la séquence de la xylanase à partir de la méthode décrite dans SAMBROOK et al. pages 13.15 et 13.17 (figure 13.3B) en utilisant le plasmide pUBR-720X11 comme matrice.

Pour initier la détermination de la séquence, des oligonucléotides synthétiques sont préparés pour s'hybrider avec le plasmide pUBR2002. Les séquences de ces oligonucléotides synthétiques sont les suivantes : et

Le reste de la séquence est déterminé par l'utilisation d'autres oligonucléotides synthétiques en se basant sur les parties de la séquence nouvellement déterminées.

La séquence de nucléotides du gène complet qui code pour la xylanase (SEQ ID NO:10) est ainsi obtenue (figure 2). Le gène de la xylanase est obtenu comme un fragment d'environ 1,5 kpb

### Exemple 15

### Construction du vecteur d'expression pUBRD-720X11

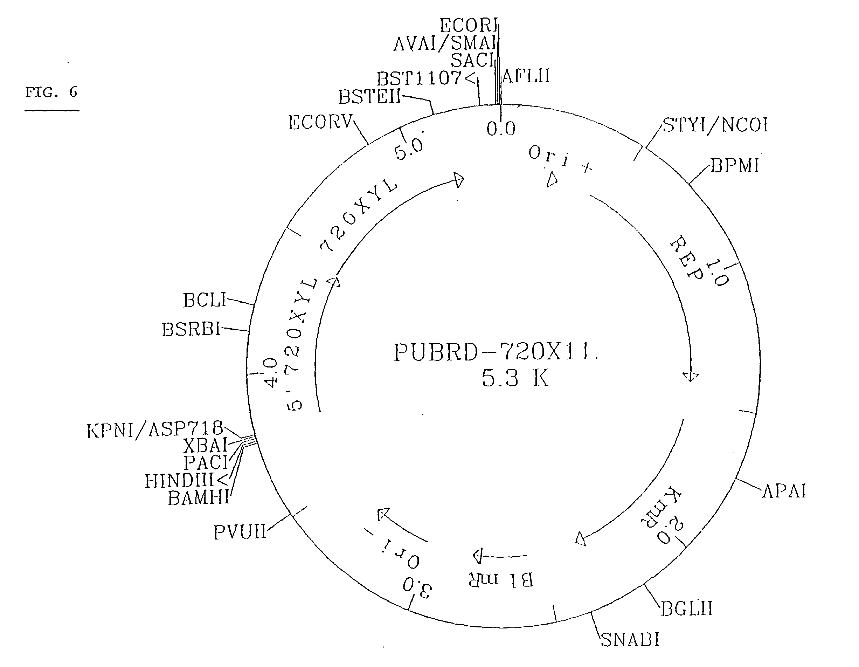

Le vecteur d'expression pUBRD-720X11 (Figure 6) est un plasmide dérivé du plasmide pUBR-720X11, dont on a éliminé le réplicon de E. coli.

La construction du plasmide pUBRD-720X11 1 à partir du plasmide pUBR-720X11, tel qu'obtenu à l'exemple 14, est décrite ci-après.

Le plasmide pUBR-720X11 est digéré par l'enzyme de restriction aux sites SnaBI, en vue d'éliminer l'origine de réplication de E. coli, en suivant la technique décrite à l'exemple 14. Un fragment d'environ 5 kpb est ainsi obtenu, il est ligaturé sur lui-même, en suivant la technique décrite à l'exemple 14, pour obtenir le plasmide pUBRD-720X11.

La ligation ainsi obtenue est utilisée pour transformer des cellules compétentes de Bacillus subtilis SE3 en suivant la technique décrite dans DNA Cloning, vol. II, ED. Glover, D.M., IRL Press Oxford, 1985, pages 9-11.

La souche de Bacillus subtilis SE3 a été déposée le 21 juin 1993 à la collection nommée BELGIAN COORDINATED COLLECTIONS OF MICROORGANISMS (LMG culture collection, Gand, Belgique) conformément au Traité de Budapest sous le numéro LMG P-14035.

Les cellules transformées sont mises en culture sur boîte de Pétri contenant le milieu LB (Luria-Bertani) gélosé, 0,8 g/l d'AZCL-xylane et 25 µg/ml de kanamycine, à 37°C pendant environ 18 heures. Après croissance, les colonies présentant une zone d'hydrolyse sont isolées.

Les colonies isolées sont soumises à une analyse du plasmide par restriction enzymatique, en vue de vérifier que la construction du plasmide est correcte, en suivant la technique décrite à l'exemple 14.

On obtient une souche dont on peut isoler le vecteur qui est dénommé pUBRD-720X11.

### Exemple 16

### Transformation de la souche de Bacillus licheniformis SE2 delap6 avec le vecteur d'expression pUBRD-720X11

Le plasmide pUBRD-720X11, décrit à l'exemple 15, est extrait de son hôte, isolé et purifié (SAMBROOK et al., 1989, p. 1.25-1.28).

On prépare une culture de la souche de Bacillus licheniformis SE2 delap6. La souche de Bacillus licheniformis SE2 delap6 et la culture de celle-ci sont décrites aux exemples 27 et 28 de la demande de brevet européen 0 634 490.

La souche de Bacillus licheniformis SE2 delap6 a été preparée à partir de la souche de Bacillus licheniformis SE2 qui a été déposée le 21 juin 1993 à la collection nommée BELGIAN COORDINATED COLLECTIONS OF MICROORGANISMS (LMG culture collection, Gand, Belgique) conformément au Traité de Budapest sous le numéro LMG P-14034.

Puis, on transforme la souche de Bacillus licheniformis SE2 delap6 avec le plasmide pUBRD-720X11 selon la technique des protoplastes (MANIATIS et al., p. 150-151).

La souche transformée [Bacillus licheniformis SE2 delap6 (pUBRD-720X11)] est sélectionnée sur boîte de Pétri contenant le milieu LB gélosé, 0,8 g/l d'AZCL-xylane et 25 *µ*g/ml de kanamycine. Puis, elle est isolée et purifiée par criblage, c'est-à-dire en étant déposée et étalée sous forme de stries épuisantes à la surface du milieu LB gélosé (Luria Bertani) qui est décrit dans Molecular Cloning - Laboratory Manual (Sambrook et al.), 1989, p. A.4.

### Exemple 17

### Production de la xylanase par B. licheniformis SE2 delap6 (pUBRD-720X11)

La souche de B. licheniformis SE2 delap6 transformée par le plasmide pUBRD-720X11, telle qu'obtenue à l'exemple 16, est mise en culture pendant 17 heures à 37°C dans un milieu de culture LB supplémente de 0,5% (p/v) de glucose et 20 µg/ml de kanamycine.

Cette culture est transférée (5 % v/v) dans 50 ml de milieu M2 supplémenté de 20 µg/ml de kanamycine.

Le milieu M2 contient 30 g de farine de soja, 75 g d'amidon soluble, 2 g de sulfate de sodium, 5 mg de chlorure de magnésium, 3 g de NaH₂PO₄, 0,2 g de CaCl₂.H₂O et 1000 ml d'eau. Le pH de ce milieu M2 est ajusté à 5,8 avec NaOH 10 N avant sa stérilisation.

La culture est incubée sous agitation orbitale, à raison de 250 révolutions par minute avec une amplitude d'environ 2,54 cm, pendant 80 heures à 37°C. Après 80 heures, la biomasse est éliminée par centrifugation (BECKMAN J21, rotor JA10) à 5000 tours par minutes pendant 10 minutes. Le surnageant de centrifugation est conservé. On mesure l'activité enzymatique sur ce surnageant, selon la technique décrite à l'exemple 2, et on note la présence d'une activité xylanase.

### Exemple 18

### Construction du vecteur pUBC2001

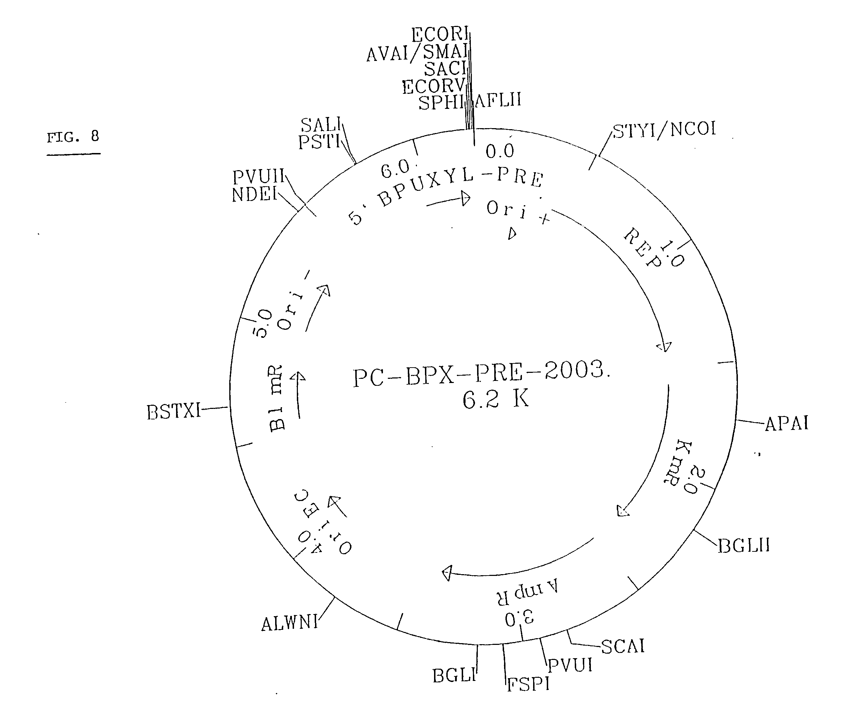

Le vecteur pUBC2001 (E. coli-Bacillus) (figure 7) est un plasmide dérivé du plasmide pUBC131, comportant comme seule différence la présence de deux sites de restriction supplémentaires : BstEII et PacI. La construction du vecteur pUBC131 est décrite à l'exemple 11 et à la figure 8 du brevet Etats-Unis 5,352,603 (demande de brevet européen 0 415 296).

La construction de ce plasmide est décrite ci-après.

On construit quatre oligonucléotides synthétiques en suivant la technique décrite à l'exemple 14.

Les séquences de ces quatre oligonucléotides sont les suivantes :

Les deux oligonucléotides ayant les séquences SEQ ID NO: 18 et 19 sont utilisés pour effectuer une amplification par PCR à partir du vecteur pUBC131, en suivant la technique PCR décrite à l'exemple 14. Le fragment amplifié par PCR, contenant une partie du gène de résistance à l'ampicilline et les fonctions nécessaires à la réplication dans B. coli, est soumis à une restriction par ScaI et BstEII, générant un fragment d'environ 1,5 - 1,6 kpb.

Une seconde amplification PCR est réalisée à partir du vecteur pUBC131, en utilisant les oligonucléotides ayant les séquences SEQ ID NO: 20 et 21 et en suivant la technique décrite à l'exemple 14. Le fragment amplifié par PCR contient une partie du vecteur pUBC131. Ce fragment est soumis à une restriction par BstEII et EcoRI, générant un fragment d'environ 1,4 - 1,5 kpb.

Les deux fragments ainsi obtenus sont ligaturés ensemble, selon la technique décrite à l'exemple 14, avec le vecteur pUBC131 qui a préalablement fait l'objet d'une double digestion avec EcoRI et ScaI, en suivant la technique décrite à l'exemple 14.

La ligation ainsi obtenue est utilisée pour transformer des cellules d'E. coli MC1061 par électroporation, en suivant la technique décrite à l'exemple 14. Les cellules transformées sont mises en culture sur boîte de Pétri contenant le milieu LB gélosé, 100 *µ*g/ml d'ampicilline et 10 *µ*g/ml de kanamycine, à 37°C pendant environ 18 heures.

Après croissance, les colonies isolées sont soumises à une analyse du plasmide par restriction enzymatique, en suivant la technique décrite à l'exemple 14.

On obtient une souche dont on peut extraire le vecteur qui est dénommé pUBC2001.

### Exemple 19

### Construction du vecteur d'expression pC-BPX-PRE 2003

Le vecteur d'expression pC-BPX-PRE-2003 (E. coli-Bacillus) (Figure 8) est un vecteur d'expression dérivé du plasmide pUBC2001. Il contient le promoteur dérivé du gène qui code pour la xylanase de Bacillus pumilus PRL B12 et la préséquence qui code pour le peptide signal de la xylanase de Bacillus pumilus PRL B12. Le procédé de préparation et d'obtention du promoteur dérivé du gène qui code pour la xylanase de Bacillus pumilus PRL B12 et la préséquence qui code pour le peptide signal de la xylanase de Bacillus pumilus PRL B12 est décrit à l'exemple 17 et à la figure 1 de la demande de brevet européen 0 634 490.

La séquence du promoteur dérivé du gène qui code pour la xylanase de Bacillus pumilus PRL B12 est décrite dans la présente demande sous le numéro SEQ ID NO: 26. La séquence de la préséquence qui code pour le peptide signal de la xylanase de Bacillus pumilus PRL B12 est décrite dans la présente demande sous le numéro SEQ ID NO: 27.

La construction du plasmide pC-BPX-PRE-2003 est décrite ci-après.

On construit deux oligonucléotides synthétiques en suivant la technique décrite à l'exemple 14.

Les séquences de ces deux oligonucléotides sont les suivantes :

Ces deux oligonucléotides sont utilisés pour effectuer une amplification par PCR à partir du plasmide pUB-BPX12, selon la technique décrite à l'exemple 14. La construction du plasmide pUB-BPX12 est décrite à l'exemple 17 et à la figure 4 de la demande de brevet européen 0 634 490, qui est incorporée par référence.

L'utilisation de l'oligonucléotide ayant la séquence SEQ ID NO: 22 permet, par le changement d'un nucléotide d'éliminer, en amont du promoteur de la xylanase de B. pumilus PRL B12, le site de restriction SphI, situé à environ 5,5 kpb, présent normalement dans pUBC2001. Le changement de nucléotide est représenté par le nucléotide souligné dans la séquence SEQ ID NO: 22 ci-avant et concerne la substitution de C par G pour le site SphI (SphI = GCATGC).

La séquence SEQ ID NO: 23 permet de créer un nouveau site SphI à la fin de la préséquence qui code pour le peptide signal de la xylanase de B. pumilus PRL B12 en changeant seulement un nucléotide du codon Ala [25], c'est-à-dire en changeant GCG en GCT.

Le fragment amplifié par PCR, contenant le promoteur dérivé du gène qui code pour la xylanase de B. pumilus PRL B12 et la préséquence qui code pour le peptide signal de la xylanase de B. pumilus PRL B12, est soumis à une restriction par PvuII et SphI, générant un fragment d'environ 0,7 kpb, en suivant la technique décrite à l'exemple 14.

Le fragment PvuII-SphI, d'environ 0,7 kpb, est ligaturé avec le vecteur pUBC2001 qui a préalablement fait l'objet d'une double digestion avec PvuII et SphI, en suivant les techniques décrites à l'exemple 14.

La ligation ainsi obtenue est utilisée pour transformer des cellules d'E. coli MC1061 par électroporation, en suivant la technique décrite à l'exemple 14. Les cellules transformées sont mises en culture sur boîte de Pétri contenant le milieu LB gélosé, 100 *µ*g/ml d'ampicilline, à 37°C pendant environ 18 heures.

Après croissance, les colonies isolées sont soumises à une analyse du plasmide par restriction enzymatique, en suivant la technique décrite à l'exemple 14.

On obtient une souche dont on peut extraire le vecteur qui est dénommé pC-BPX-PRE-2003.

### Exemple 20

### Construction du vecteur d'expression pC-BPX-PRE-720X

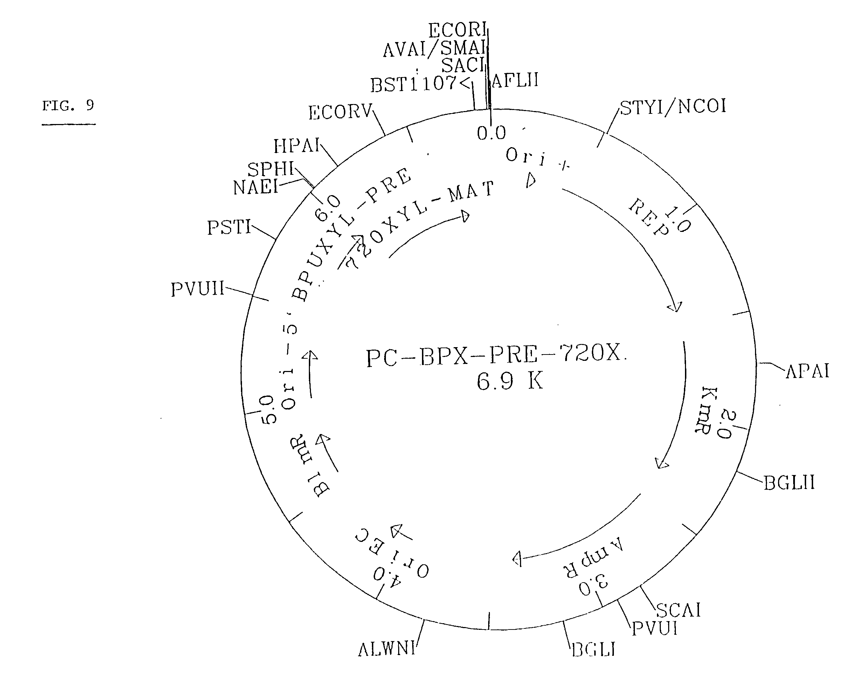

Le vecteur d'expression pC-BPX-PRE-720X (E. coli-Bacillus) (Figure 9) est un vecteur d'expression comportant le promoteur dérivé du gène qui code pour la xylanase de B. pumilus PRL B12 et la préséquence qui code pour le peptide signal de la xylanase de B. pumilus PRL B12 et la séquence du gène qui code pour la partie mature de la xylanase de Bacillus sp. 720/1.

Le vecteur d'expression pC-BPX-PRE-720X comporte la séquence SEQ ID NO:1, séquence de nucléotides du gène qui code pour la partie mature de la xylanase de Bacillus sp. 720/1.

La construction du plasmide pC-BPX-PRE-720X est décrite ci-après.

On construit deux oligonucléotides synthétiques en suivant la technique décrite à l'exemple 14.

Les séquences de ces deux oligonucléotides sont les suivantes :

Ces deux oligonucléotides sont utilisés pour effectuer une amplification par PCR à partir du plasmide pUBR-720X11, tel qu'obtenu à l'exemple 14, et selon la technique décrite à l'exemple 14.

Le fragment amplifié par PCR, contenant la séquence du gène qui code pour la partie mature de la xylanase de Bacillus sp. 720/1, est soumis à une restriction par SphI et SacI, générant un fragment d'environ 0,8 kpb, selon la technique décrite à l'exemple 14.

Le fragment SphI-SacI est ligaturé avec le vecteur pC-BPX-PRE-2003 qui a préalablement fait l'objet d'une double digestion avec SphI et SacI, selon les techniques décrites à l'exemple 14. La ligature au niveau du site de restriction SphI permet de créer une fusion traductionnelle de la séquence signal du gène qui code pour la xylanase de B. pumilus PRL B12 avec la séquence du gène qui code pour la partie mature de la xylanase de Bacillus sp. 720/1.

La ligation ainsi obtenue est utilisée pour transformer des cellules d'E. coli MC1061 par électroporation, selon la technique décrite à l'exemple 14. Les cellules transformées sont mises en culture sur boîte de Pétri contenant le milieu LB gélosé, 0,8 g/l d'AZCL-xylane et 100 µg/ml d'ampicilline, à 37°C pendant environ 18 heures. On isole les colonies présentant une zone d'hydrolyse.

Après croissance, les colonies isolées sont soumises à une analyse du plasmide par restriction enzymatique, selon la technique décrite à l'exemple 14.

On obtient une souche dont on peut extraire le vecteur qui est dénommé pC-BPX-PRE-720X.

### Exemple 21

### Construction du vecteur pBPXD-PRE-720X

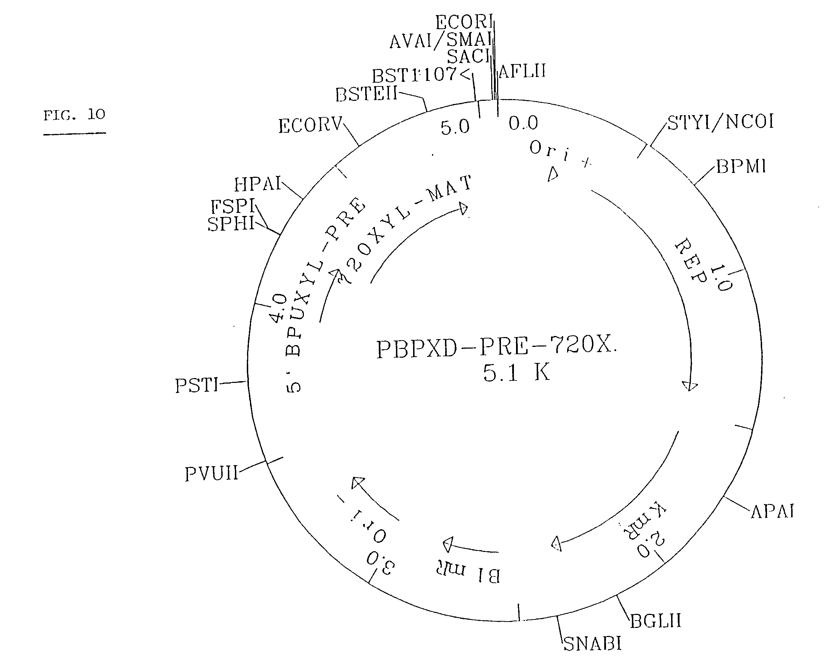

Le vecteur pBPXD-PRE-720X (Bacillus) (Figure 10) est un vecteur d'expression dérivé du plasmide pUB131. Il comporte le promoteur dérivé du gène qui code pour la xylanase de B. pumilus PRL B12 et la préséquence qui code pour le peptide signal de la xylanase de B. pumilus PRL B12 et la séquence du gène qui code pour la partie mature de la xylanase de Bacillus sp. 720/1.

La construction du plasmide pBPXD-PRE-720X est décrite ci-après.

Le plasmide pC-BPX-PRE-720X, obtenu à l'exemple 20, est soumis à une restriction par PvuII et EcoRI, générant un fragment d'environ 1,5 kpb, selon la technique décrite à l'exemple 14.

Le fragment d'environ 1,5 kpb est ligaturé avec le vecteur pUB131, qui a préalablement fait l'objet d'une double digestion avec PvuII et EcoRI, selon les techniques décrites à l'exemple 14.

La ligation ainsi obtenue est utilisée pour transformer des cellules de B. subtilis SE3, selon la technique d'électroporation décrite à l'exemple 14. Les cellules transformées sont mises en culture sur boîte de Pétri contenant le milieu LB gélosé, 0,8 g/l d'AZCL-xylane et 25 µg/ml de kanamycine, à 37°C pendant environ 18 heures. On isole les colonies présentant une large zone d'hydrolyse.

Après croissance, les colonies isolées sont soumises à une analyse du plasmide par restriction enzymatique, selon la technique décrite à l'exemple 14.

On obtient ainsi une souche dont on peut extraire le vecteur qui est dénommé pBPXD-PRE-720X.

### Exemple 22

### Transformation de Bacillus licheniformis SE2 delap6 avec le vecteur d'expression pBPXD-PRE-720X

Le plasmide pBPXD-PRE-720X (Figure 10), décrit à l'exemple 21, est extrait de son hôte, isolé et purifié.

On prépare une culture de la souche de B. licheniformis SE2 delap6, selon la technique décrite à l'exemple 16. Puis, on transforme cette souche avec ce plasmide selon la technique des protoplastes décrite à l'exemple 16.

La souche transformée [B. licheniformis SE2 delap6 (pBPXD-PRE-720X)] est sélectionnée sur boîte de Pétri contenant le milieu LB gélosé, 0,8 g/l d'AZCL-xylane et 25 *µ*g/ml de kanamycine. La souche est isolée et purifiée par criblage, selon la technique décrite à l'exemple 16.

### Exemple 23

### Production de la xylanase par B. licheniformis SE2 delap6 IpBPXD-PRE-720X)

On effectue un essai identique à celui décrit à l'exemple 17, mais avec la souche de B. licheniformis SE2 delap6 transformée par le plasmide pBPXD-PRE-720X, telle qu'obtenue à l'exemple 22.

On mesure, selon la technique décrite à l'exemple 2, l'activité enzymatique sur le surnageant obtenu et on note la présence d'une activité xylanase.

### Exemple 24

### Préparation et isolement de la xylanase produite par la souche de B. licheniformis SE2 delap6 (pBPXD-PRE-720X)

On isole et purifie la xylanase produite par la souche de B. licheniformis SE2 delap6 transformée par le plasmide pBPXD-PRE-720X, telle qu'obtenue à l'exemple 22. On met en culture cette souche en suivant le protocole décrit dans l'exemple 23.

La xylanase obtenue est isolée et purifiée suivant le protocole décrit à l'exemple 3 de la demande de brevet européen 0 634 490.

### Exemple 25

### Séquence d'acides aminés de la xylanase produite par la souche de B. licheniformis SE2 delap6 (pBPXD-PRE-720X)

La séquence des 50 premiers acides aminés de la xylanase produite par la souche de B. licheniformis SE2 delap6 (pBPXD-PRE-720X) est déterminée en utilisant un appareil séquenceur (HP G1000A, HEWLETT-PACKARD) et en suivant la notice d'emploi de cet appareil.

On met en oeuvre la xylanase isolée et purifiée telle que décrite à l'exemple 24.

On vérifie que la séquence obtenue est identique à celle décrite à l'exemple 4 pour la xylanase produite par la souche de Bacillus sp. 720/1.

### Exemple 26

### Détermination du poids moléculaire de la xylanase produite par la souche de B. licheniformis SE2 delap6 (pBPXD-PRE-720X)

Le poids moléculaire de la xylanase produite par la souche de B. licheniformis SE2 delap6 (pBPXD-PRE-720X) est déterminé en suivant le protocole décrit à l'exemple 7 et en mettant en oeuvre la xylanase isolée et purifiée telle que décrite à l'exemple 24.

Une coloration au bleu de Coomassie fait apparaître un polypeptide d'un poids moléculaire compris entre 25 et 26 kD, qui est identique à celui de la xylanase produite par la souche de Bacillus sp. 720/1.

## Revendications

1. Xylanase isolée et purifiée, **caractérisée en ce qu'**elle comprend la séquence d'acides aminés de 1 à 221 acides aminés donnés à la SEQ ID NO: 3.

2. Xylanase selon la revendication 1, **caractérisée en ce qu'**elle est un précurseur contenant 248 acides aminés donnés à la SEQ ID NO: 6.

3. Une culture isolée et purifiée de Bacillus sp. 720/1 (LMG P-14798).

4. Molécule d'ADN comprenant la séquence de nucléotides donnée à la SEQ ID NO 1 qui code pour la xylanase mature selon la revendication 1 de Bacillus sp. 720/1 (LMG P-14798).

5. Molécule d'ADN selon la revendication 4, **caractérisée en ce qu'**elle comprend la séquence de nucléotides donnée à la SEQ ID NO: 4 qui code pour le précurseur de la xylanase de Bacillus sp. 720/1 (LMG P-14798).

6. Molécule d'ADN selon la revendication 4 ou 5, **caractérisée en ce qu'**elle comprend tout le gène donné à la SEQ ID NO: 10 de la xylanase de Bacillus sp. 720/1 (LMG P-14798).

7. Molécule d'ADN selon la revendication 4, caratérisée en ce qu'elle comprend le promoteur donné à la SEQ ID NO: 26 dérivé du gène qui code pour la xylanase de Bacillus pumilus PRL B12 ATCC 55443.

8. Molécule d'ADN selon la revendication 4, **caractérisée en ce qu'**elle comprend la préséquence donnée à la SEQ ID NO: 27 qui code pour le peptide signal de la xylanase de Bacillus pumilus PRL B12 ATCC 55443.

9. Molécule d'ADN selon la revendication 7 ou 8, **caractérisée en ce qu'**elle comprend le promoteur donné à la SEQ ID NO: 26 dérivé du gène qui code pour la xylanase de Bacillus pumilus PRL B12 ATCC 55443, la préséquence donnée à la SEQ ID NO: 27 qui code pour le peptide signal de la xylanase de Bacillus pumilus PRL B12 ATCC 55443 et la séquence de nucléotides donnée à la SEQ ID NO: 1 qui code pour la xylanase selon la revendication 1 ou 2 de Bacillus sp. 720/1 (LMG P-14798).

10. Vecteur d'expression ou vecteur d'intégration chromosomique contenant la molécule d'ADN selon la revendication 4, 5, 6 ou 9.

11. Vecteur d'expression selon la revendication 10 pUBRD-720X11 dont la carte est donnée à la figure 6.

12. Vecteur d'expression selon la revendication 11 pBPXD-PRE-720X dont la carte est donnée à la figure 10.

13. Souche transformée comprenant la molecule d'ADN selon la revendication 4, 5, 6 ou 9.

14. Souche transformée comprenant le vecteur d'expression ou le vecteur d'intégration chromosomique selon la revendication 10, 11 ou 12.

15. Souche transformée selon la revendication 13 ou 14, **caractérisée en ce qu'**il s'agit d'une souche de Bacillus.

16. Souche transformée selon la revendication 15, **caractérisée en ce qu'**il s'agit d'une souche de Bacillus licheniformis ou de Bacillus pumilus.

17. Procédé pour la production d'une xylanase selon la revendication 1 ou 2 **caractérisée en ce qu'**il comprend la culture d'une souche selon l'une quelconque des revendications 13 à 16, dans un milieu nutritif approprié contenant des sources de carbone et d'azote et des sels minéraux sous condition d'aérobiose et la récolte de la xylanase ainsi obtenue.

18. Procédé selon la revendication 17 pour la préparation d'une xylanase selon la revendication 1 ou 2 **caractérisée en ce qu'**il comprend l'isolement d'un fragment d'ADN codant pour la xylanase, l'insertion de ce fragment d'ADN dans un vecteur approprié, l'introduction de ce vecteur dans un hôte approprié ou l'introduction de ce fragment d'ADN dans le chromosome d'un hôte approprié, la culture de cet hôte, l'expression de la xylanase et la récolte de la xylanase.

19. Utilisation d'une xylanase selon la revendication 1 ou 2 pour le traitement de la pâte à papier.

20. Utilisation d'une xylanase selon la revendication 1 ou 2 en alimentation animale.

21. Une composition enzymatique contenant une xylanase selon revendication 1 ou 2 et au moins un additif.

22. Système d'expression utilisable pour la production d'un polypeptide, **caractérisé en ce qu'**il comprend:
- la séquence du promoteur donné à la SEQ ID NO: 26 dérivé du gène qui code pour la xylanase de Bacillus pumilus PRL B12 ATCC 55443,
- une séquence codant pour un signal peptide, et
- la séquence de nucléotides donnée à la SEQ ID NO: 1 qui code pour la xylanase de Bacillus sp. 720/1 (LMG P-14798).

23. Système d'expression utilisable pour la production d'un polypeptide, **caractérisé en ce qu'**il comprend:
- la séquence d'un promoteur,
- la préséquence donnée à la SEQ ID NO:27 qui code pour le peptide signal de la xylanase de Bacillus pumilus PRL B12 ATCC 55443, et
- la séquence de nucléotides donnée à la SEQ ID NO: 1 qui code pour la xylanase de Bacillus sp. 720/1 (LMG P-14798).

24. Système d'expression utilisable pour la production d'un polypeptide, **caractérisé en ce qu'**il comprend:
- la séquence du promoteur donnée à la SEQ ID NO: 26 dérivé du gène qui code pour la xylanase de Bacillus pumilus PRL B12 ATCC 55443,
- la préséquence donnée à la SEQ ID NO: 27 qui code pour le peptide signal de la xylanase de Bacillus pumilus PRL B12 ATCC 55443,
- la séquence de nucléotides donnée à la SEQ ID NO: 1 qui code pour la xylanase de Bacillus sp. 720/1 (LMG P-14798), et
- la séquence d'un terminateur.

## Patentansprüche

1. Isolierte und gereinigte Xylanase, **dadurch gekennzeichnet, dass** sie die Aminosäuresequenz der die Aminosäuren 1 bis 221 von Seq.-ID Nr. 3 umfasst.

2. Xylanase nach Anspruch 1, **dadurch gekennzeichnet, dass** sie ein Vorläufer ist, der die 248 Aminosäuren von Seq.-ID Nr. 6 umfasst.

3. Isolierte und gereinigte Kultur von Bacillus sp. 720/1 (LMG P-14798).

4. DNA-Molekül, umfassend die Nucleotidsequenz mit der Seq.-ID Nr. 1, die für eine reife Xylanase nach Anspruch 1 von Bacillus sp. 720/1 (LMG P-14798) kodiert.

5. DNA-Molekül nach Anspruch 4, **dadurch gekennzeichnet, dass** es die Nucleotidsequenz mit der Seq.-ID Nr. 4 umfasst, die für den Vorläufer der Xylanase von Bacillus sp. 720/1 (LMG P-14798) kodiert.

6. DNA-Molekül nach Anspruch 4 oder 5, **dadurch gekennzeichnet, dass** es das gesamte Gen mit der Seq.-ID Nr. 10 für die Xylanase von Bacillus sp. 720/1 (LMG P-14798) umfasst.

7. DNA-Molekül, nach Anspruch 4, **dadurch gekennzeichnet, dass** es den Promotor mit der Seq.-ID Nr. 26 umfasst, der von dem für die Xylanase von Bacillus pumilus PRL B12 ATCC 55443 kodierenden Gen abgeleitet ist.

8. DNA-Molekül nach Anspruch 4, **dadurch gekennzeichnet, dass** es die Präsequenz mit der Seq.-ID Nr. 27 umfasst, die für das Signalpeptid der Xylanase von Bacillus pumilus PRL B12 ATCC 55443 kodiert.

9. DNA-Molekül nach Anspruch 7 oder 8, **dadurch gekennzeichnet, dass** es den Promotor mit der Seq.-ID Nr. 26, der von dem für die Xylanase von Bacillus pumilus PRL B12 ATCC 55443 kodierenden Gen abgeleitet ist, die Präsequenz mit der Seq.-ID Nr. 27, die für das Signalpeptid der Xylanase von Bacillus pumilus PRL B12 ATCC 55443 kodiert, und die Nucleotidsequenz mit der Seq.-ID Nr. 1, die für die Xylanase von Bacillus sp. 720/1 (LMG P-14798) nach Anspruch 1 oder 2 kodiert, umfasst.

10. Expressionsvektor oder Chromosom-Integrationsvektor, umfassend ein DNA-Molekül nach Anspruch 4, 5, 6 oder 9.

11. Expressionsvektor pUBRD-720X11 nach Anspruch 10, dessen Karte in Fig. 6 gezeigt ist.

12. Expressionsvektor pBPXD-PRE-720X nach Anspruch 11, dessen Karte in Fig. 10 gezeigt ist.

13. Transformierter Stamm, umfassend ein DNA-Molekül nach Anspruch 4, 5, 6 oder 9.

14. Transformierter Stamm, umfassend einen Expressionsvektor oder Chromosom-Integrationsvektor nach Anspruch 10, 11 oder 12.

15. Transformierter Stamm nach Anspruch 13 oder 14, **dadurch gekennzeichnet, dass** es sich um einen Bacillus-Stamm handelt.

16. Transformierter Stamm nach Anspruch 15, **dadurch gekennzeichnet, dass** es sich um einen Stamm von Bacillus licheniformis oder Bacillus pumilus handelt.

17. Verfahren zur Produktion einer Xylanase nach Anspruch 1 oder 2, **dadurch gekennzeichnet, dass** es das Kultivieren eines Stamms nach einem der Ansprüche 13 bis 16 in einem geeigneten Nährmedium, das Kohlenstoff- und Stickstoffquellen und Mineralsalze enthält, unter aeroben Bedingungen, sowie das Gewinnen der so erhaltenen Xylanase umfasst.

18. Verfahren nach Anspruch 17 zur Herstellung einer Xylanase nach Anspruch 1 oder 2, **dadurch gekennzeichnet, dass** es das Isolieren eines DNA-Fragments, das für die Xylanase kodiert, die Insertion des DNA-Fragments in einen geeigneten Vektor, die Einführung des Vektors in einen geeigneten Wirt oder die Einführung des DNA-Fragments in das Chromosom eines geeigneten Wirts, das Kultivieren des Wirts, das Exprimieren der Xylanase und das Gewinnen der Xylanase umfasst.

19. Verwendung einer Xylanase nach Anspruch 1 oder 2 zur Behandlung von Papiermasse.

20. Verwendung einer Xylanase nach Anspruch 1 oder 2 für Futtermittel.

21. Enzymzusammensetzung, umfassend eine Xylanase nach Anspruch 1 oder 2 und zumindest ein Additiv.

22. Expressionssystem, das für die Produktion eines Polypeptids geeignet ist, **dadurch gekennzeichnet, dass** es Folgendes umfasst:
- die Sequenz des Promotors mit der Seq.-ID Nr. 26, der von dem für die Xylanase von Bacillus pumilus PRL B12 ATCC 55443 kodierenden Gen abgeleitet ist,
- eine Sequenz, die für ein Signalpeptid kodiert, und
- die Nucleotidsequenz mit der Seq.-ID Nr. 1, die für die Xylanase von Bacillus sp. 720/1 (LMG P-14798) kodiert.

23. Expressionssystem, das für die Produktion eines Polypeptids geeignet ist, **dadurch gekennzeichnet, dass** es Folgendes umfasst:
- die Sequenz eines Promotors,
- die Präsequenz mit der Seq.-ID Nr. 27, die für das Signalpeptid der Xylanase von Bacillus pumilus PRL B12 ATCC 55443 kodiert, und
- die Nucleotidsequenz mit der Seq.-ID Nr. 1, die für die Xylanase von Bacillus sp. 720/1 (LMG P-147998) kodiert.

24. Expressionssystem, das für die Produktion eines Polypeptids geeignet ist, **dadurch gekennzeichnet, dass** es Folgendes umfasst:
- die Sequenz des Promotors mit der Seq.-ID Nr. 26, der von dem für die Xylanase von Bacillus pumilus PRL B12 ATCC 55443 kodierenden Gen abgeleitet ist,
- die Präsequenz mit der Seq.-ID Nr. 27, die für das Signalpeptid der Xylanase von Bacillus pumitus PRL B12 ATCC 55443 kodiert,
- die Nucleotidsequenz mit der Seq.-ID Nr. 1, die für die Xylanase von Bacillus sp. 720/1 (LMG P-14798) kodiert, und
- eine Terminatorsequenz.

## Claims

1. Isolated and purified xylanase, **characterized in that** it comprises the amino acid sequence from 1 to 221 amino acids given in SEQ ID NO:3

2. Xylanase according to Claim 1, **characterized in that** it is a precursor containing 248 amino acids given in SEQ ID NO:6.

3. Isolated and purified culture of Bacillus sp. 720/1 (LMG P-14798).

4. DNA molecule comprising the nucleotide sequence given in SEQ ID NO:1 which codes for the mature xylanase according to Claim 1 of Bacillus sp. 720/1 (LMG P-14798).

5. DNA molecule according to Claim 4, **characterized in that** it comprises the nucleotide sequence given in SEQ ID NO:4. which codes for the Bacillus sp. 720/1 xylanase precursor (LMG P-14798).

6. DNA molecule according to Claim 4 or 5, **characterized in that** it comprises the entire Bacillus sp. 720/1 (LMGP-14798) xylanase gene given in SEQ ID NO:10.

7. DNA molecule according to Claim 4, **characterized in that** it comprises the promoter given in SEQ ID NO:26 derived from the gene which codes for Bacillus pumilus PRL B12 ATCC 55443 xylanase.

8. DNA molecule according to Claim 4, **characterized in that** it comprises the presequence given in SEQ ID NO:27 which codes for the signal peptide of Bacillus pumilus PRL B12 ATCC 55443 xylanase.

9. DNA molecule according to Claim 7 or 8, **characterized in that** it comprises the promoter given in SEQ ID NO:26 derived from the gene which codes for Bacillus pumilus PRL B12 ATCC 55443 xylanase, the presequence given in SEQ ID NO:27 which codes for the signal peptide of Bacillus pumilus PRL B12 ATCC 55443 xylanase and the nucleotide sequence given in SEQ ID NO:1 which codes for the Bacillus sp. 720/1 (LMG P-14798) xylanase according to Claim 1 or 2.

10. Expression vector or chromosomal integration vector containing the DNA molecule according to Claim 4, 5, 6 or 9.

11. Expression vector according to Claim 10 pUBRD-720X11 for which the map is shown in Figure 6.

12. Expression vector according to Claim 11 pBPXD-PRE-720X for which the map is shown in Figure 10.

13. Transformed strain comprising the DNA molecule according to Claim 4, 5, 6 or 9.

14. Transformed strain comprising the expression vector or the chromosomal integration vector according to Claim 10, 11 or 12.

15. Transformed strain according to Claim 13 or 14, **characterized in that** it is a Bacillus strain.

16. Transformed strain according to Claim 15, **characterized in that** it is a Bacillus licheniformis or Bacillus pumilus strain.

17. Method for the production of a xylanase according to Claim 1 or 2, **characterized in that** it comprises the culturing of a strain according to any one of Claims 13 to 16 in a suitable nutrient medium containing carbon and nitrogen sources and inorganic salts under aerobic conditions, and the harvesting of the xylanase thereby obtained.

18. Method according to Claim 17 for the preparation of a xylanase according to Claim 1 or 2, **characterized in that** it comprises isolation of a DNA fragment coding for the xylanase, the insertion of this DNA fragment into a suitable vector, the introduction of this vector into a suitable host or the introduction of this DNA fragment into the chromosome of a suitable host, the culturing of this host, the expression of the xylanase and the harvesting of the xylanase.

19. Use of a xylanase according to Claim 1 or 2, for the treatment of paper pulp.

20. Use of a xylanase according to Claim 1 or 2 in animal feeds.

21. Enzyme composition containing a xylanase according to Claim 1 or 2, and at least one additive.

22. Expression system which can be used for the production of a polypetide, **characterized in that** it comprises:
- the sequence of the promoter given in SEQ ID NO:26 derived from the gene which codes for Bacillus pumilus PRL B12 ATCC 55443 xylanase,
- a sequence coding for a signal peptide, and
- the nucleotide sequence given in SEQ ID NO:1 which codes for Bacillus sp. 720/1 (LMG P-14798) xylanase.

23. Expression system which can be used for the production of a polypeptide, **characterized in that** it comprises:
- the sequence of a promoter,
- the presequence given in SEQ ID NO:27 which codes for the signal peptide of Bacillus pumilus PRL B12 ATCC 55443 xylanase, and
- the nucleotide sequence given in SEQ ID NO:1 which codes for Bacillus sp. 720/1 (LMG P-14798) xylanase.

24. Expression system which can be used for the production of a polypeptide, **characterized in that** it comprises:
- the sequence of the promoter given in SEQ ID NO:26 derived from the gene which codes for Bacillus pumilus PRL B12 ATCC 55443 xylanase, the presequence given in SEQ ID NO:27 which codes for the signal peptide of Bacillus pumilus PRL B12 ATCC 55443 xylanase,
- the nucleotide sequence given in SEQ ID NO:1 which codes for Bacillus sp. 720/1 (LMG P-14798) xylanase, and .
- the sequence of a terminator.
